# EUROPÄISCHE PATENTSCHRIFT

(11) **EP 2 612 538 B1**
(45) Veröffentlichungstag und Bekanntmachung des Hinweises auf die Patenterteilung: **07.12.2016**
(21) Anmeldenummer: 11767158.6
(22) Anmeldetag: 02.09.2011
(51) Int. Cl.: H05B 3/26, A61M 1/16, A61M 5/44

(54) **ELEKTRISCH ÜBER EINEN NETZSPANNUNGSANSCHLUSS BETREIBBARES GERÄT**
APPARATUS THAT CAN BE ELECTRICALLY OPERATED VIA A MAINS VOLTAGE CONNECTION
APPAREIL ÉLECTRIQUE POUVANT FONCTIONNER SUR SECTEUR

(30) Priorität: 03.09.2010 DE 102010036295
(43) Veröffentlichungstag der Anmeldung: 10.07.2013
(73) Patentinhaber: Fresenius Medical Care Deutschland GmbH, 61352 Bad Homburg (DE)
(72) Erfinder: SEBESTA, Sven, 97421 Schweinfurt (DE); WERNICKE, Ulrich, 97531 Theres (DE)
(74) Vertreter: Laufhütte, Dieter
(86) Internationale Anmeldenummer: PCT/EP2011/004438
(87) Internationale Veröffentlichungsnummer: WO 2012/028329

(56) Entgegenhaltungen:
- EP-A2- 1 623 733
- EP-B1- 1 222 087
- EP-B1- 1 263 549
- WO-A1-2009/044220
- WO-A2-03/099355
- DE-A1- 4 108 804
- DE-A1- 4 442 825
- US-A- 5 760 488
- US-A1- 2003 217 962

## Beschreibung

Die vorliegende Erfindung betrifft ein elektrisch über einen Netzspannungsanschluss betreibbares medizinisches Gerät mit einem spannungsführenden Element und einem Anwendungsteil, wobei das Anwendungsteil gegenüber dem spannungsführenden Element durch eine Basisisolation isoliert ist. Insbesondere handelt es sich dabei um ein Gerät ohne Schutzleiteranschluss. Die vorliegende Erfindung betrifft insbesondere ein Dialysegerät mit einem Heizmodul zum Erwärmen einer medizinischen Flüssigkeit, wobei das Heizmodul ein Heizelement mit einem auf einer ersten keramischen Schicht angeordneten Heizwendel umfasst.

Werden Geräte an der Netzspannung betrieben, so muss verhindert werden, dass Anwender des Gerätes durch einen elektrischen Schlag gefährdet werden. Insbesondere muss dabei verhindert werden, dass ein gefährlicher elektrischer Strom durch den menschlichen Körper fließt, wenn ein Anwender mit dem Anwendungsteil in Berührung kommt. Hierfür ist zunächst eine Basisisolation zwischen den spannungsführenden Elementen des Gerätes und dem Anwendungsteil vorgesehen, welche gemäß der Einteilung der Schutzmaßnahmen als Basisschutz dient. Neben einem solchen Basisschutz wird jedoch weiterhin ein Fehlerschutz gefordert. Ein solcher Fehlerschutz soll verhindern, dass bei einem Ausfall des Basisschutzes, das heißt bei einer mangelhaften Isolation zwischen dem spannungsführenden Element und dem Anwendungsteil eine Gefährdung für den Anwender auftreten kann. Als Fehlerschutz sind dabei die Verwendung eines Schutzleiters, das heißt die Erdung des Gerätes, oder der Einsatz eines Trenntransformators zur galvanischen Entkopplung der spannungsführenden Teile von der Netzspannung bekannt. Ein Schutzleitetanschluß steht jedoch nicht überall zur Verfügung, und ein Trenntransformator führt zu einer Erhöhung von Bauraum, Gewicht und Kosten. Besonders relevant wird diese Problematik bei Geräten mit einem großen Ohmschen Verbraucher wie beispielsweise einem Heizmodul. Solche Heizmodule werden dabei insbesondere bei medizinischen Geräten, wie z. B. einem Dialysegerät eingesetzt, um eine medizinische Flüssigkeit zu erwärmen. Solche Heizmodule weisen üblicherweise ein Heizelement mit einem auf einer ersten keramischen Schicht angeordneten Heizwendel auf, wobei der Heizwendel über die Netzspannung mit Strom versorgt wird.

Die Dokumenten EP 1 623 733 A2, WO 2009/044220 A1 und DE 41 08 804 A1 beschreiben medizinische Geräten aus dem Stand der Technik. Dokument DE 44 42 825 A1 offenbart ein System zum Speichern elektrischer Energie für ein Elektrofahrzeug mit einer Basisisolation und einem Isolationswächter.

Aufgabe der vorliegenden Erfindung ist es daher, ein an einem Netzspannungsanschluss betreibbares medizinisches

Gerät zur Verfügung zu stellen, welches neben dem Basisschutz durch eine Basisisolation einen weiteren Fehlerschutz aufweist. Weiterhin ist es Aufgabe der vorliegenden Erfindung, ein medizinisches Gerät mit einem verbesserten Heizmodul zur Verfügung zu stellen.

Diese Aufgaben werden erfindungsgemäß durch ein Geräte gemäß Anspruch 1 oder ein Verfahren gemäß Anspruch 13 gelöst.Vorteilhafte Ausgestaltungen der vorliegenden Erfindung sind Gegenstand der Unteransprüche.

Die vorliegende Erfindung umfasst in einem ersten Aspekt ein elektrisch über einen Netzspannungsanschluss betreibbares medizinisches Gerät mit einem spannungsführenden Element und einem Anwendungsteil, wobei das Anwendungsteil gegenüber dem spannungsführenden Element durch eine Basisisolation isoliert ist. Erfindungsgemäß ist dabei ein Isolationswächter vorgesehen, welcher die Qualität der Basisisolation des Anwendungsteiles gegenüber dem spannungsführenden Element überwacht. Insbesondere überwacht der Isolationswächter dabei die Güte, d.h. den Übergangswiderstand der Basisisolation. Durch den Isolationswächter ist es möglich, ständig die Qualität der Basisisolation gegenüber dem Anwenderteil zu überwachen und den Anwender so vor einem gefährlichen elektrischen Stromschlag zu schützen, wenn die Basisisolation ausfallen sollte. Die Überwachung durch den Isolationswächter kann damit als zweite Schutzmaßnahme gewertet werden und erlaubt es, das Gerät ohne Schutzleiter zu betreiben. Insbesondere kommt die vorliegende Erfindung daher bei solchen Geräten zum Einsatz, welche keinen Schutzleiter aufweisen. Der sonst durch den Schutzleiter zur Verfügung gestellte Fehlerschutz wird damit durch den Isolationswächter zur Verfügung gestellt.

Isolationswächter werden in der Technik bisher nur bei sogenannten IT-Systemen eingesetzt, bei welchen ein abgrenzbarer Bereich eines Stromnetzes durch einen Trenntransformator vom übrigen Stromnetz getrennt wird, wobei auf eine Erdung des abgetrennten Bereiches des Stromnetzes verzichtet wird. Die bei solchen IT-Systemen eingesetzten Isolationswächter überwachen dabei die Qualität der Isolation des Netzes gegenüber einem Erdanschluss. Verschlechtert sich diese Qualität, werden üblicherweise Warnhinweise gegeben. Da der abgetrennte Bereich des Stromnetzes jedoch nicht geerdet ist, geht von nur einem Fehler in der Isolation noch keine akute Gefahr aus, so dass eine Abschaltung des Netzes nicht notwenig ist. Andere Einsatzmöglichkeiten für Isolationswächter waren aus dem Stand der Technik dagegen bisher nicht bekannt.

Anders als im Stand der Technik wird durch den erfindungsgemäßen Isolationswächter nicht die Isolation zwischen einem Stromnetz und einem Erdanschluß überwacht, sondern die Qualität der Basisisolation eines Anwendungsteils des Gerätes gegenüber einem spannungsführenden Element des Gerätes. Zudem kann das erfindungsgemäße Gerät auch an einem Netzspannungsanschluss betrieben werden, welcher geerdet ist.

Erfindungsgemäß handelt es sich bei dem Gerät um ein medizinisches Gerät. Der Isolationswächter erlaubt es dabei, dieses medizinische Gerät ohne Schutzleiter zu betreiben, so dass dieses vielseitiger einsetzbar wird. Insbesondere handelt es sich erfindungsgemäß um ein Dialysegerät.

Weiterhin ist erfindungsgemäß vorgesehen, dass der Isolationswächter einen Stromfluss und/oder Widerstand zwischen dem spannungsführenden Element und dem Anwendungsteil bestimmt. Dabei kann der Isolationswächter sowohl auf einem passiven, als auch auf einem aktiven Messprinzip beruhen. Bei einem passiven Messprinzip bestimmt der Isolationswächter lediglich passiv einen Stromfluss zwischen dem spannungsführenden Element und dem Anwendungsteil. Aus der Größe dieses Stromflusses lässt sich die Qualität der Basisisolation bestimmen. Bei einer aktiven Meßmethode legt der Isolationswächter dagegen ein Spannungssignal zwischen dem spannungsführenden Element und dem Anwendungsteil an und bestimmt den daraus resultierenden Stromfluss. Hieraus lässt sich der Widerstand der Basisisolation und damit deren Qualität bestimmen. Insbesondere steht der Isolationswächter hierfür über Verbindungsleitungen mit dem spannungsführenden Element und dem Anwendungsteil elektrisch leitend in Verbindung. Insbesondere kann dabei erfindungsgemäß der Stromfluss und/oder Widerstand zwischen dem Anwendungsteil und mindestens einer Spannungszuführung des spannungsführenden Elementes bestimmt werden.

Vorteilhafterweise ist vorgesehen, dass der Isolationswächter jeweils den Stromfluss und/oder Widerstand zwischen dem Anwendungsteil und einer ersten und den Stromfluss und/oder Widerstand zwischen dem Anwendungsteil und einer zweiten Spannungszuführung des spannungsführenden Elementes bestimmt. Durch die Messung des Stromflusses und/oder des Widerstands zwischen dem Anwendungsteil und den beiden Spannungszuführungen lässt sich die Qualität der Basisisolation sicher bestimmen.

Vorteilhafterweise kommt die vorliegende Erfindung bei solchen Geräten zum Einsatz, bei welchen das spannungsführende Element ohne eine galvanische Trennung an der Netzspannung betrieben wird. Insbesondere kann durch den Isolationswächter auf eine solche galvanische Trennung z. B. durch einen Trenntransformator verzichtet werden, ohne die Sicherheit eines Anwenders zu gefährden.

Vorteilhafterweise schaltet der Isolationswächter die Stromversorgung des spannungsführenden Elementes erfindungsgemäß ab, wenn er eine defekte Basisisolation erkennt. Überschreitet daher der Stromfluss oder unterschreitet der Widerstand zwischen dem spannungsführenden Element und dem Anwendungsteil einen gewissen Grenzwert, so schließt der Isolationswächter hieraus auf eine defekte Basisisolierung und schaltet die Stromversorgung des spannungsführenden Elementes ab, um den Anwender vor einem gefährlichen elektrischen Stromfluss durch seinen Körper bei Berührung des Anwendungsteils zu schützen. Vorteilhafterweise werden dabei beide Spannungszuführungen des spannungsführenden Elementes unterbrochen.

Alternativ oder zusätzlich zu dem Abschalten der Stromversorgung für das spannungsführende Element kann der Isolationswächter vorteilhafterweise die Gerätesteuerung informieren, dass die Basisisolation fehlerhaft ist. Insbesondere kann dabei eine Anzeige erfolgen, welche die Bedienperson auf die Fehlerhaftigkeit der Basisisolation aufmerksam macht. Alternativ kann auch die Stromversorgung des gesamten Gerätes abgeschaltet werden.

Weiterhin vorteilhafterweise weist die Gerätesteuerung des Gerätes eine Funktion zum Testen der ordnungsgemäßen Funktion des Isolationswächters auf. Insbesondere kann dabei bei Inbetriebnahme des Gerätes ein Initialtest durchgeführt werden, welcher die ordnungsgemäße Funktion des Isolationswächters sicherstellt. Hierfür kann die Basisisolierung über einen Schalter und einen Widerstand überbrückt werden und dabei überprüft werden, ob der Isolationswächter dies erkennt.

Die vorliegende Erfindung kann insbesondere bei einem Gerät eingesetzt werden, bei welchem das spannungsführende Element einen Verbraucher mit einem relativ hohen Stromverbrauch darstellt. Bei solchen Verbrauchern würde ein Trenntransformator eine entsprechend große Dimensionierung benötigen, auf welche nun verzichtet werden kann. Insbesondere weist das spannungsführende Element dabei einen Verbrauch von mehr als 100 W, weiterhin vorteilhafterweise von mehr als 500 W auf.

Insbesondere kann es sich bei dem spannungsführenden Element gemäß der vorliegenden Erfindung um ein Heizelement handeln. Insbesondere kann es sich dabei um ein keramisches Heizelement handeln. Bei einem solchen Heizelement ist eine Widerstandsbahn auf einer Keramikplatte angeordnet. Durch Anlegen einer Spannung an der Widerstandsbahn erhitzt sich diese und gibt die Wärme an die Keramikplatte ab. Die Keramikplatte dient dabei gleichzeitig als Basisisolation. Die vorliegende Erfindung kann jedoch auch bei anderen spannungsführenden Elementen eingesetzt werden, z. B. bei Motoren oder ähnlichem.

Bei dem Anwendungsteil des erfindungsgemäßen Gerätes handelt es sich insbesondere um ein Element des Gerätes, welches ein Anwender bei der Bedienung des Gerätes berühren könnte. Weiterhin handelt es sich insbesondere um ein elektrisch leitendes Element bzw. ein Element aus Metall.

Weiterhin kann es sich bei dem Anwendungsteil um ein Gehäuseelement handeln. Insbesondere handelt es sich bei dem Anwendungsteil dabei um eine Heizplatte, welche über ein Heizelement erwärmt und durch die Basisisolation von diesem getrennt ist. Insbesondere kann es sich bei einer solchen Heizplatte um eine Metallplatte, insbesondere um eine Aluminiumplatte handeln.

Insbesondere kommt die vorliegende Erfindung dabei in einem Dialysegerät zum Einsatz, wobei das spannungsführende Element ein keramisches Heizelement und das Anwendungsteil eine Heizplatte darstellt, welche von der Keramikschicht des keramischen Heizelementes gegeneinander isoliert sind. Über dieses Heizelement kann vorteilhafterweise das Dialysat beheizt werden. Insbesondere ist dabei ein Heizbereich des Fluidsystems an die Heizplatte ankoppelbar. Der erfindungsgemäße Isolationswächter erlaubt dabei den Betrieb eines solchen Dialysegeräts ohne einen Schutzleiteranschluss.

Die vorliegende Erfindung umfasst weiterhin ein Verfahren zum Betrieb eines elektrischen Gerätes über einen Netzspannungsanschluss, zum Betrieb eines medizinischen Gerätes, wobei das Gerät ein spannungsführendes Element und ein Anwendungsteil aufweist und wobei das Anwendungsteil von dem spannungsführenden Element durch eine Basisisolation isoliert ist. Erfindungsgemäß ist dabei vorgesehen, dass die Qualität der Basisisolation des Anwendungsteiles gegenüber dem spannungsführenden Element überwacht wird. Insbesondere wird dabei ständig die Qualität der Basisisolation überwacht und bei einer Überschreitung eines Grenzwertes die Abschaltung der Stromversorgung des spannungsführenden Elementes vorgenommen.

Vorteilhafterweise wird das erfindungsgemäße Verfahren so durchgeführt, wie dies bereits oben hinsichtlich des erfindungsgemäßen Gerätes beschrieben wurde. Insbesondere handelt es sich bei dem erfindungsgemäßen Verfahren dabei um ein Verfahren zum Betrieb eines elektrischen Gerätes, wie es oben beschrieben wurde.

In einem zweiten nicht beanspruchten Aspekt umfasst die vorliegende Erfindung ein medizinisches Gerät mit einem Heizmodul zum Erwärmen einer medizinischen Flüssigkeit, wobei das Heizmodul ein Heizelement mit einem auf einer ersten keramischen Schicht angeordneten Heizwendel umfasst. Erfindungsgemäß ist dabei vorgesehen, dass das Heizelement auf einer zweiten keramischen Schicht angeordnet ist. Durch die zweite keramische Schicht, auf welcher das Heizelement erfindungsgemäß angeordnet wird, wird so durch den konstruktiven Aufbau des Heizmoduls eine zweite Isolierung zur Verfügung gestellt, welche neben der durch die erste keramische Schicht gebildeten Basisisolierung für einen verbesserten Schutz des Anwenders sorgt.

Insbesondere kann es sich bei dem medizinischen Gerät um ein Dialysegerät handeln, wobei das Heizmodul zum Erwärmen von Dialysat und/oder Blut eingesetzt wird. Insbesondere kann es sich dabei um ein Peritonealdialysegerät handeln, bei welchem das Heizmodul zum Erwärmen des Dialysats eingesetzt wird.

Vorteilhafterweise kann die zweite keramische Schicht, welche gemäß dem zweiten Aspekt der vorliegenden Erfindung vorgesehen ist, die Heizplatte des Heizmoduls bilden. Vorteilhafterweise können an diese Heizplatte die Fluidwege des medizinischen Geräts angekoppelt werden, um die in den Fluidwegen befindliche medizinische Flüssigkeit zu erwärmen.

Bei dem Heizwendel handelt es sich dabei vorteilhafterweise um eine Widerstandsbahn, welche auf der ersten keramischen Schicht aufgebracht ist. Vorteilhafterweise sind dabei Anschlüsse zum Anschließen des Heizwendels an die Versorgungsspannung vorgesehen.

Vorteilhafterweise wird das erfindungsgemäße medizinische Gerät dabei über einen Netzspannungsanschluß betrieben. Insbesondere kann es sich dabei um ein Gerät ohne einen Schutzleiteranschluß handeln. Durch die durch die zweite keramische Schicht zur Verfügung gestellte weitere Isolation kann dabei die Heizung dennoch ohne einen Trenntransformator betrieben werden.

In einer bevorzugten Ausführung der vorliegenden Erfindung bestehen die erste und/oder die zweite keramische Schicht aus Aluminiumoxid oder aus Aluminiumnitrid. Beide Materialien haben sehr gute Wärmeleiteigenschaften und sind gleichzeitig hervorragende elektrische Isolatoren. In einer besonders bevorzugten Ausführungsform besteht dabei die erste keramische Schicht aus Aluminiumoxid, während die zweite keramische Schicht aus Aluminiumnitrid besteht.

Bevorzugt ist das Heizelement mit der ersten keramischen Schicht auf die zweite keramische Schicht aufgeklebt. Insbesondere erfolgt die Verklebung dabei großflächig über die gesamte Fläche des Heizelementes. Durch die Verwendung eines Klebers zur Verbindung der keramischen Schicht des Heizelementes mit der zweiten keramischen Schicht können unterschiedliche Ausdehnungskoeffizienten der für die beiden Schichten verwendeten Materialien ausgeglichen werden.

Vorteilhafterweise handelt es sich bei dem Kleber dabei um einen Wärmeleitkleber, insbesondere um einen Silikonklebstoff.

In einer bevorzugten Ausführungsform bildet sowohl die erste keramische Schicht, als auch die zweite keramische Schicht eine Platte. Vorteilhafterweise ist dabei die die zweite keramische Schicht bildende Platte größer als die die erste keramische Schicht bildende Platte. So kann die die zweite keramische Schicht bildende Platte als ein Trägerelement für das Heizelement mit der ersten keramischen Schicht bilden.

Vorteilhafterweise weist die die zweite keramische Schicht bildende Platte keine Durchbrechungen in dem Bereich auf, in welchem auf der ersten keramischen Schicht der Heizwendel angeordnet ist. Vorteilhafterweise weist die zweite keramische Schicht in den Bereichen, in welchen die erste keramische Schicht vorgesehen ist, überhaupt keine Durchbrechungen auf. Weiterhin vorteilhafterweise weist die die zweite keramische Schicht bildende Platte überhaupt keine Durchbrechungen auf.

Weiterhin vorteilhafterweise weist auch die erste keramische Schicht keine Durchbrechungen im Bereich der Heizwendel auf und besonders vorteilhafterweise überhaupt keine Durchbrechungen.

Durch die möglichst druchbrechungsfreie Ausgestaltung der keramischen Schichten wird die Isolationssicherheit erhöht.

Weiterhin können auf dem Heizelement des Heizmodules einer oder mehrere Temperatursensoren vorgesehen sein. Insbesondere kann dabei auf der gleichen Seite der ersten keramischen Schicht, auf welcher auch der Heizwendel angeordnet ist, ein Temperatursensor angeordnet sein.

Die erste keramische Schicht kann bevorzugt eine Dicke zwischen 0,5 mm und 2 mm aufweisen, weiterhin vorteilhafterweise eine Dicke zwischen 0,8 und 1,2 mm.

Die zweite keramische Schicht weist vorteilhafterweise eine Dicke zwischen 1 mm und 3mm auf, weiterhin vorteilhafterweise zwischen 1,2 mm und 1,8 mm.

Die Schichtdicke der Klebeschicht, welche die beiden keramischen Schichten miteinander verbindet, kann zwischen 0,01 mm und 1 mm betragen, vorteilhafterweise zwischen 0,1 mm und 0,5 mm.

Die bei der vorliegenden Erfindung eingesetzten Heizelemente können dabei vorteilhafterweise eine Maximalleistung zwischen 100 W und 2000 W aufweisen, weiterhin vorteilhafterweise zwischen 200 und 1000 W.

In einer besonderen Ausführungsform der vorliegenden Erfindung sind mindestens zwei Heizelemente vorgesehen, welche auf einer gemeinsamen zweiten keramischen Schicht angeordnet sind. Vorteilhafterweise bildet die zweite keramische Schicht dabei wiederum eine Heizplatte, auf welcher so nebeneinander zwei Heizelemente mit ihren jeweiligen ersten keramischen Schichten angeordnet sind. Vorteilhafterweise entspricht der Aufbau des Heizmoduls und der Heizelemente ansonsten der Ausführung, wie sie oben dargestellt wurde.

Die beiden Heizelemente sind dabei vorteilhafterweise über eine Leitung mit integrierter Temperatursicherung rückseitig miteinander elektrisch verbunden. Insbesondere können die beiden Heizelemente dabei in Serie geschaltet sein.

In einer weiteren vorteilhaften Ausgestaltung der vorliegenden Erfindung weist das Heizmodul weiterhin einen Rahmen auf. Insbesondere kann dabei die zweite keramische Schicht auf einen solchen Rahmen aufgeklebt sein, um die Stabilität des Heizmoduls zu erhöhen.

Weiterhin kann vorgesehen sein, dass das Heizmodul im medizinischen Gerät einvulkanisiert ist. Hierdurch ergibt sich eine besonders gute Dichtigkeit.

Die vorliegende Erfindung umfasst weiterhin ein Heizmodul für ein medizinisches Gerät, wie es oben beschrieben wurde. Insbesondere umfasst das Heizmodul dabei ein Heizelement mit einem auf einer ersten keramischen Schicht angeordneten Heizwendel, wobei das Heizelement auf einer zweiten keramischen Schicht angeordnet ist. Insbesondere handelt es sich bei der zweiten keramischen Schicht dabei um die Heizplatte des Heizmoduls. Vorteilhafterweise ist das Heizmodul dabei so aufgebaut, wie dies bereits oben im Hinblick auf das medizinische Gerät näher dargestellt wurde.

Der zweite Aspekt der vorliegenden Erfindung, bei welchem zur besseren Isolation eine zweite keramische Schicht eingesetzt wird, kann dabei erfindungsgemäß auch mit dem ersten Aspekt der vorliegenden Erfindung kombiniert werden, bei welchem ein Isolationswächter vorgesehen ist. Hierdurch ergibt sich eine nochmals höhere Sicherheit.

Die vorliegende Erfindung wird nun anhand von Ausführungsbeispielen sowie Zeichnungen näher dargestellt.

Bei dem erfindungsgemäßen elektrisch über einen Netzspannungsanschluss betreibbaren Gerät und dem erfindungsgemäßen medizinischen Gerät handelt es sich in einem vorteilhaften Ausführungsbeispiel um ein Dialysegerät. Der Aufbau eines solchen Dialysegerätes, bei welchem die vorliegende Erfindung eingesetzt wird, wird daher zunächst anhand der Figuren 1 bis 13 allgemein näher dargestellt. Ausführungsbeispiele des ersten Aspekts der vorliegenden Erfindung werden dann mit Bezug auf Figuren 14 bis 16 näher dargestellt, ein Ausführungsbeispiel des zweiten Aspekts der vorliegenden Erfindung mit Bezug auf Figur 17.

Dabei zeigen:

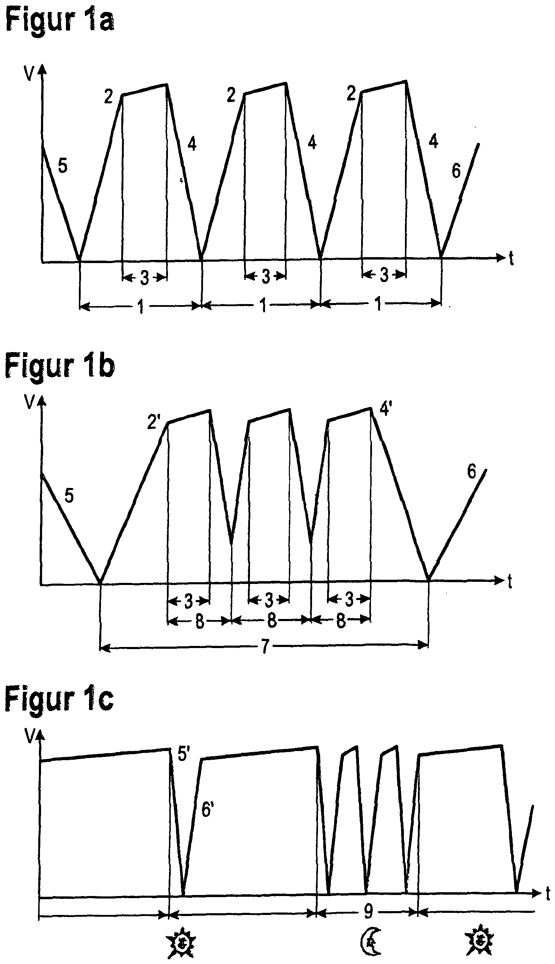

- Fig. 1: drei Diagramme, welche typische Verläufe einer automatischen Peritonealdialysebehandlung zeigen,

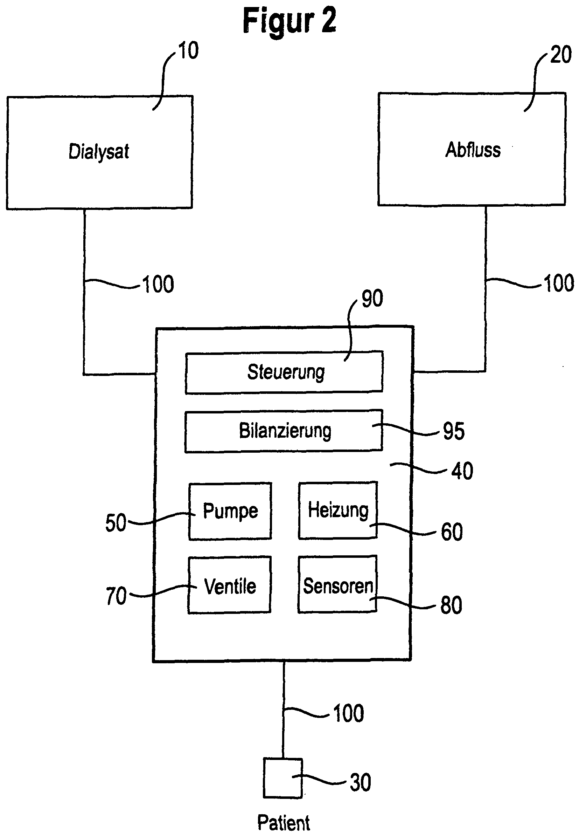

- Fig. 2: eine Prinzipdarstellung eines Peritonealdialysesystems,

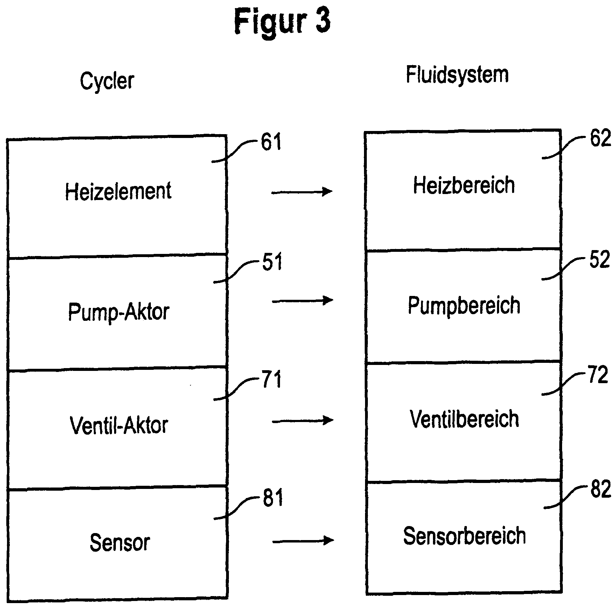

- Fig. 3: eine Prinzipdarstellung der Aufteilung des Peritonealdialysesystems in eine Dialysemaschine und ein Fluidsystem,

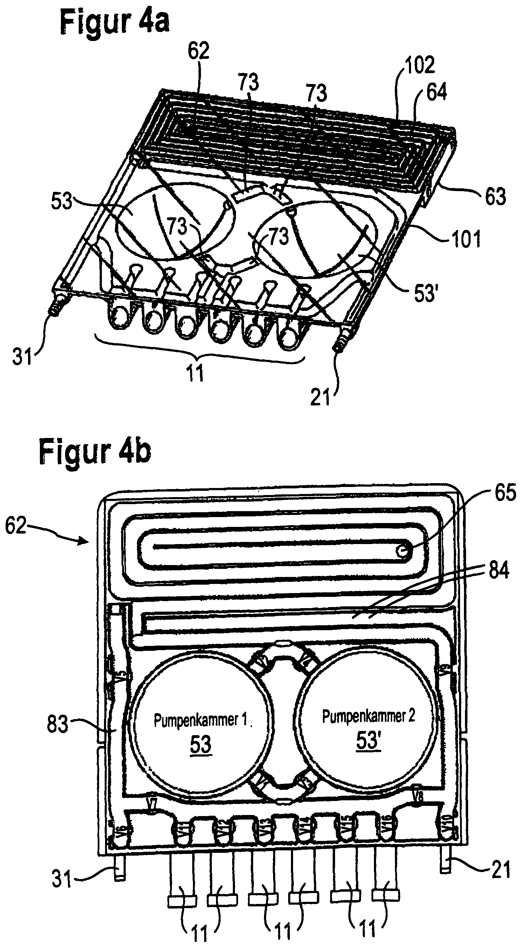

- Fig. 4: ein erstes Ausführungsbeispiel einer Kassette,

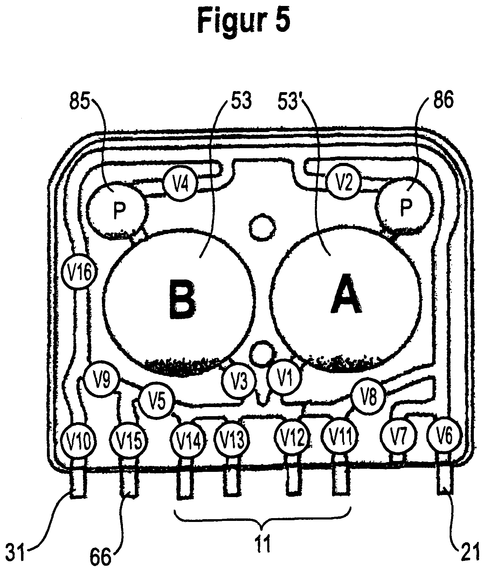

- Fig. 5: ein zweites Ausführungsbeispiel einer Kassette,

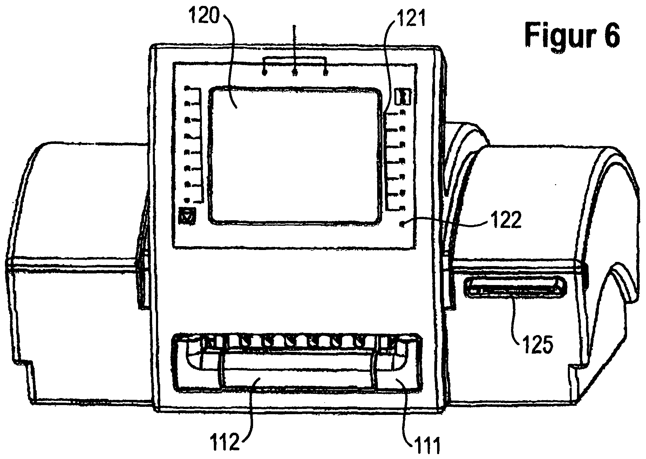

- Fig. 6: eine perspektivische Ansicht eines ersten Ausführungsbeispiels einer Dialysemaschine,

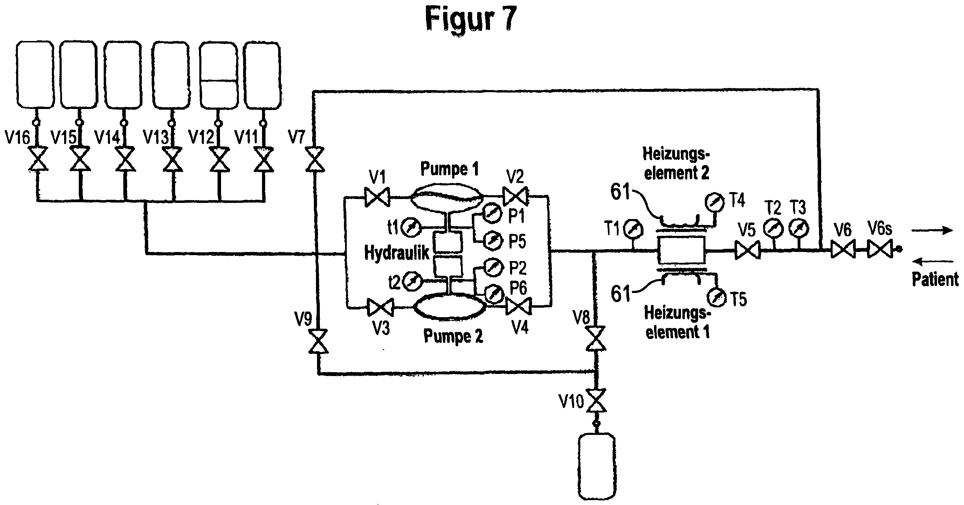

- Fig. 7: ein Flussdiagramm eines ersten Ausführungsbeispiels eines Peritonealdialysesystems,

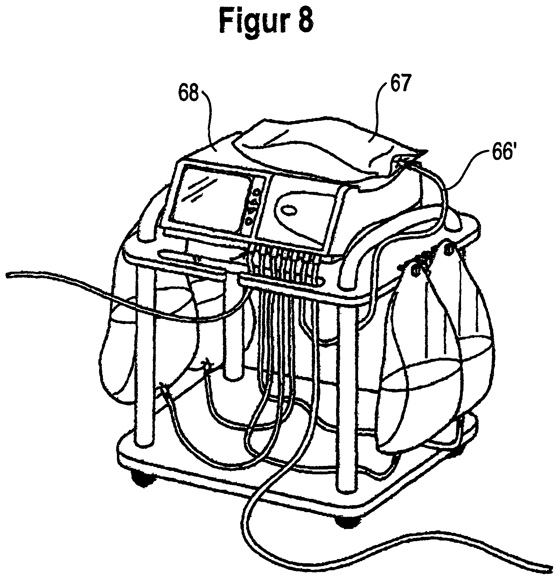

- Fig. 8: eine perspektivische Ansicht eines zweiten Ausführungsbeispiels einer Dialysemaschine,

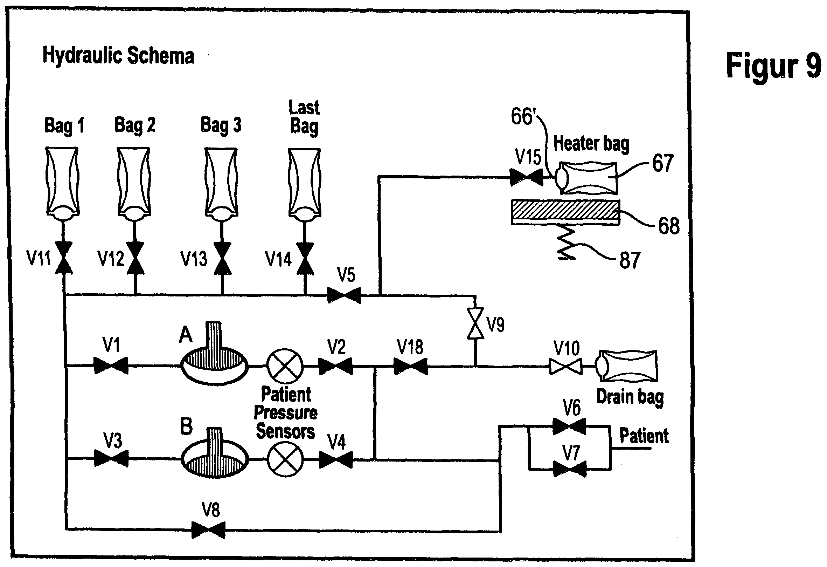

- Fig. 9: ein Flussdiagramm eines zweiten Ausführungsbeispiels eines Peritonealdialysesystems,

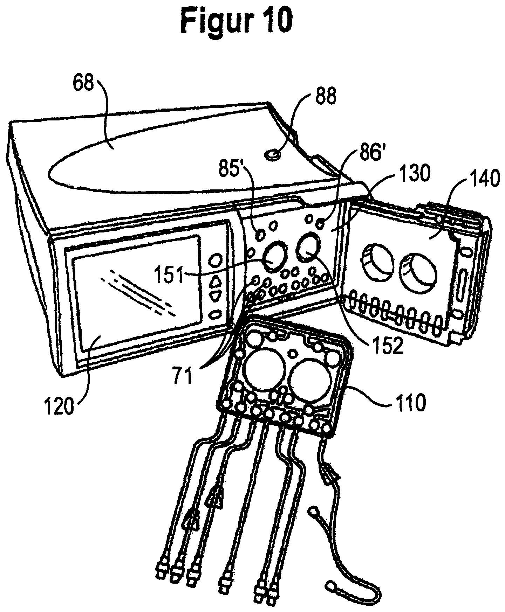

- Fig. 10: das Ankoppeln der Kassette beim zweiten Ausführungsbeispiels eines Peritonealdialysesystems,

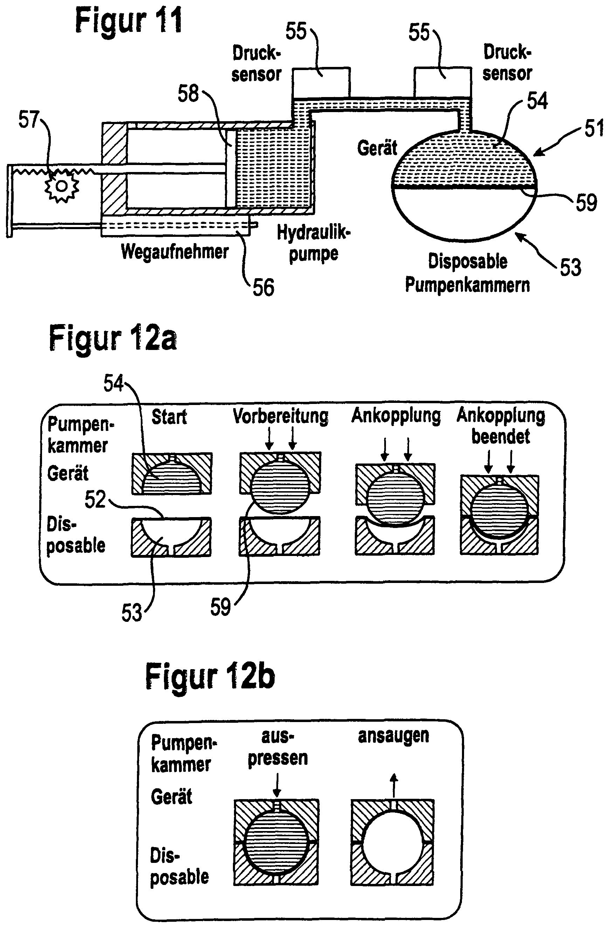

- Fig. 11: ein erstes Ausführungsbeispiels eines Pumpaktors,
- Fig. 12: das Ankoppeln eines Pumpbereichs der Kassette an einen Pumpaktor,

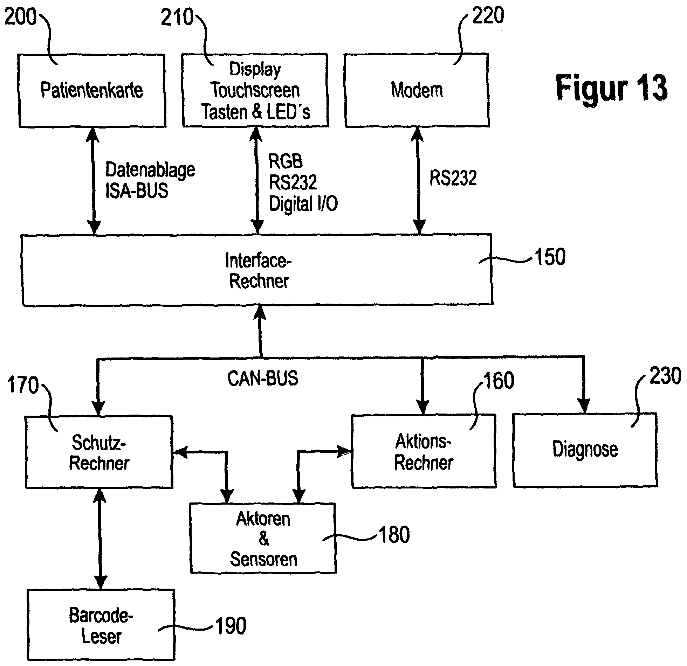

- Fig. 13: eine Prinzipdarstellung des Aufbaus eines Ausführungsbeispiels einer Steuerung, und

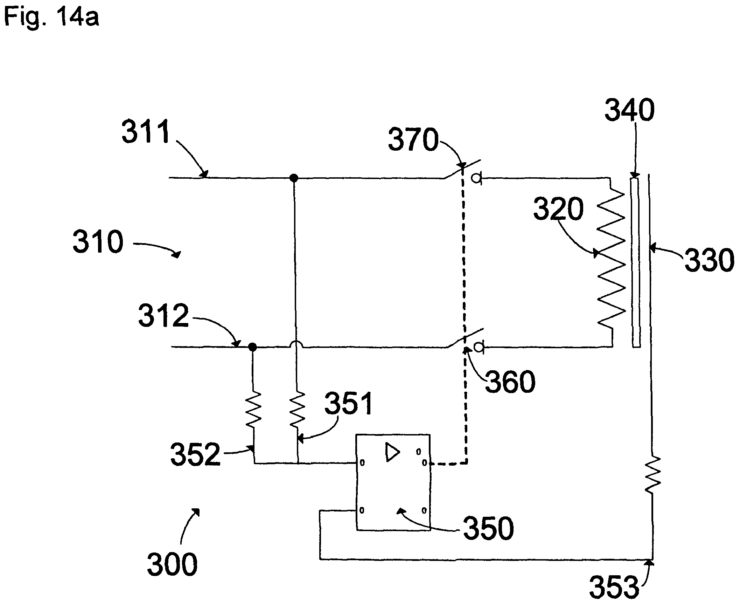

- Figur 14a:: ein Prinzipschaubild eines erfindungsgemäßen elektrisch über einen Neutzspannungsanschluss betreibbaren Gerätes mit einem Isolationswächter,

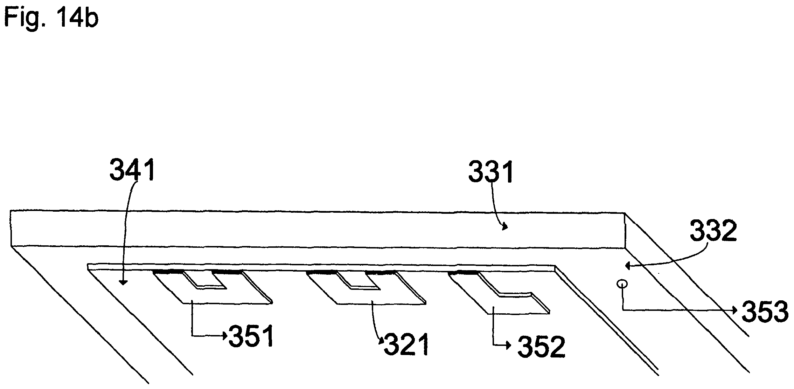

- Fig. 14b:: eine teilweise Schnittansicht durch ein Ausführungsbeispiel eines spannungsführenden Elementes eines erfindungsgemäßen Gerätes,

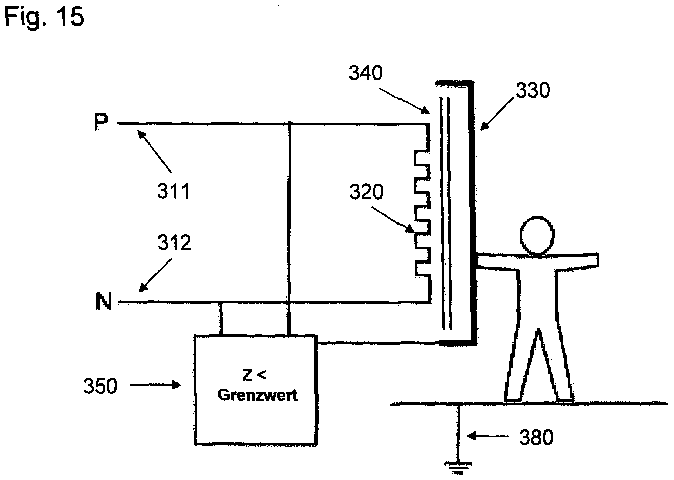

- Figur 15:: ein erstes Ausführungsbeispiel eines erfindungsgemäßen Gerätes mit einem Isolationswächter,

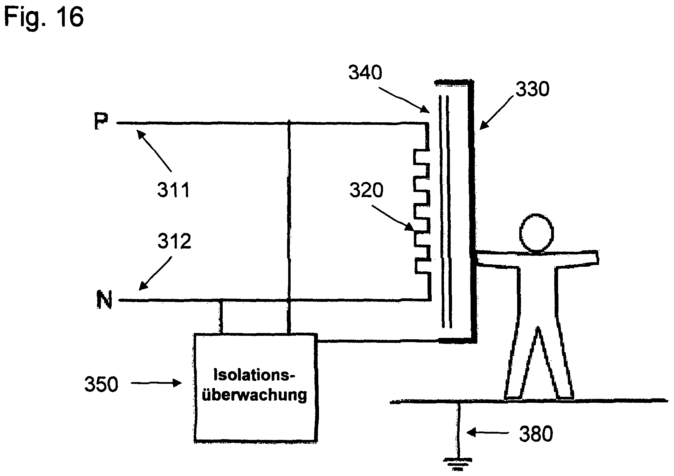

- Figur 16:: ein zweites Ausführungsbeispiel eines erfindungsgemäßen Gerätes mit einem Isolationswächter und

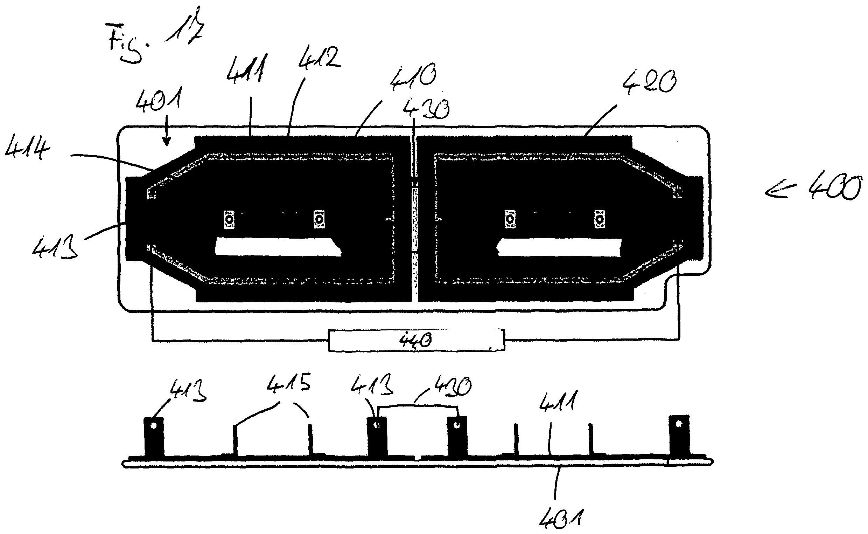

- Figur 17:: ein Ausführungsbeispiel eines erfindungsgemäßen medizinischen Gerätes gemäß dem zweiten Aspekt der vorliegenden Erfindung mit einem Heizelement, wie es auf einer zweiten keramischen Schicht angeordnet ist.

Im folgenden soll die Funktion einer Dialysemaschine, bei welcher die vorliegende Erfindung zum Einsatz kommt, zunächst allgemein beschrieben werden. Bei der Dialysemaschine handelt es sich dabei im Ausführungsbeispiel um eine Peritonealdialysemaschine. Die unten beschriebenen Komponenten können jedoch in gleicher oder ähnlicher Weise auch für eine Hämodialysemaschine eingesetzt werden.

Die Peritonealdialyse ist eine Variante der künstlichen Blutwäsche, bei welcher das gut durchblutete Bauchfell (Peritoneum) des Patienten als körpereigene Filtermembran verwendet wird. Hierfür wird über einen Katheter Dialysat in die Bauchhöhle eingeführt. Nach dem Prinzip der Osmose diffundieren nun Harnbestandteile des Blutes durch das Bauchfell in das in der Bauchhöhle befindliche Dialysat. Nach einer gewissen Verweilzeit wird das Dialysat mit den Harnbestandteilen wieder aus der Bauchhöhle ausgelassen.

Bei der automatischen Peritonealdialyse steuert und überwacht eine Dialysemaschine die Einführung des frischen Dialysats in die Bauchhöhle und das Auslassen des verbrauchten Dialysats. Eine solche Dialysemaschine, auch Cycler genannt, befüllt und entleert die Bauchhöhle dabei üblicherweise mehrmals über Nacht, d.h. während der Patient schläft.

In Figuren 1a bis 1c sind drei unterschiedliche Verfahrensabläufe gezeigt, wie sie von einer Dialysemaschine durchgeführt werden. Einer oder mehrerer dieser Verfahrensabläufe ist dabei üblicherweise in der Steuerung der Dialysemaschine hinterlegt. Dabei ist es üblicherweise möglich, die abgespeicherten Verfahrensabläufe an den Patienten anzupassen.

Bei den Figuren 1a bis 1c ist jeweils die in der Bauchhöhle des Patienten befindliche Dialysatmenge V über die Zeit t aufgezeichnet. Figur 1a zeigt dabei den Verlauf einer normalen automatischen Peritonealdialysebehandlung über Nacht. Zu Beginn der Behandlung erfolgt dabei zunächst ein initialer Auslauf 5, durch welchen Dialysat, das über den Tag in der Bauchhöhle des Patienten belassen worden war, entnommen wird. Daraufhin folgen mehrere Behandlungszyklen 1, in Figur 1a drei aufeinander folgende Behandlungszyklen 1. Jeder Behandlungszyklus besteht dabei aus einer Einlaufphase 2, einer Verweilphase 3 und einer Auslaufphase 4. Während der Einlaufphase 2 wird dabei ein gewisses Volumen an frischer Dialyseflüssigkeit in die Bauchhöhle des Patienten eingelassen. Die maximal zulässige Dialysatmenge beträgt dabei je nach Patienten zwischen ca. 1,5 und 3 1. Das frische Dialysat verbleibt nun über eine gewisse Verweilzeit 3 in der Bauchhöhle. Typischerweise dauert die Verweilphase dabei einige Stunden. Daraufhin wird das nun verbrauchte Dialysat in der Auslaufphase 4 wieder aus der Bauchhöhle herausgelassen. Daraufhin startet ein neuer Behandlungszyklus. Die Behandlung wird mit einem letzten Einlauf 6 abgeschlossen, durch welchen eine gewissen Menge an frischem Dialysat in die Bauchhöhle des Patienten eingeführt wird. Diese verbleibt dann über den Tag in der Bauchhöhle des Patienten.

Die einzelnen Behandlungszyklen 1, welche über Nacht erfolgen, werden dabei automatisch von der Steuerung der Dialysemaschine angesteuert. Der initiale Auslauf und der letzte Einlauf können ebenfalls automatisch von der Dialysemaschine angesteuert werden. Alternativ werden diese manuell von einer Bedienperson oder vom Patienten aktiviert.

In Figur 1b ist eine sogenannte Tidalbehandlung gezeigt. Auch diese beginnt mit einem initialen Auslauf 5 und endet mit einem letzten Einlauf 6. Weiterhin ist ein Basiszyklus 7 vorgesehen, welcher sich in mehrere Tidalzyklen 8 aufteilt. Dabei ist zunächst eine Basiseinlaufphase 2' vorgesehen. Nach der Verweilphase 3 wird jedoch nicht mehr das komplette Dialysatvolumen aus der Bauchhöhle entnommen, sondern nur eine gewisse Teilmenge des in der Bauchhöhle befindlichen Dialysats. Dieses wird dann durch ein entsprechendes Volumen an frischem Dialysat ersetzt. Nach einem erneuten Verweilzyklus kann eine weitere Tidalentnahme erfolgen, bei welcher nicht das gesamte im Bauchraum befindliche Dialysat entfernt wird. Zum Ende des Basiszyklus 7 erfolgt eine Basisauslaufphase 4', bei welcher nun das gesamte Dialysat entnommen wird. In Figur 1b ist dabei lediglich ein Basiszyklus 1 dargestellt. Alternativ können jedoch auch mehrere Basiszyklen vorgesehen sein.

In Figur 1c ist der Verlauf einer Peritonealdialysebehandlung mit einer sogenannten PD-Plus-Behandlung gezeigt. Dabei erfolgt während der Nacht 9 eine übliche Peritonealdialysebehandlung, welche z. B. gemäß den Figuren 1 a oder 1 b durchgeführt werden kann. Weiterhin ist jedoch während des Tages eine zusätzliche PD-Plus-Behandlung vorgesehen, bei welcher in einer Auslaufphase 5' das verbrauchte Dialysat entnommen und in einer Einlaufphase 6' durch frisches Dialysat ersetzt wird. Bei der PD-Plus-Behandlung wird damit eine normale nächtliche Peritonealdialysebehandlung mit einem oder mehreren zusätzlichen Behandlungszyklen während des Tages kombiniert. Der Verlauf der nächtlichen Behandlung wird dabei wie üblich automatisch durch die Dialysemaschine durchgeführt. Die Behandlungszyklen während des Tages werden ebenfalls über die Maschine durchgeführt und überwacht.

In Figur 2 ist nun der Aufbau eines typischen Peritonealdialysesystems schematisch dargestellt. Das Peritonealdialysesystem umfasst dabei einen Behälter 10 mit frischem Dialysat und einen Abfluss 20 für gebrauchtes Dialysat. Weiterhin ist ein Konnektor 30 vorgesehen, welcher an einen Katheter des Patienten angeschlossen werden kann, um entweder frisches Dialysat in die Bauchhöhle des Patienten einzuführen oder gebrauchtes Dialysat aus der Bauchhöhle abzuführen. Der Behälter 10 mit frischem Dialysat, der Abfluss 20 für gebrauchtes Dialysat und der Konnektor 30 zum Patienten sind dabei über Fluidwege 100 miteinander verbunden und bilden zusammen mit diesen das Fluidsystem des Peritonealdialysesystems.

Zur Durchführung der Peritonealdialysebehandlung ist eine Dialysemaschine 40, auch Cycler genannt, vorgesehen. Die Dialysemaschine 40 umfasst dabei folgende Hauptkomponenten:
Eine Pumpe 50, welche zum Transport der Flüssigkeiten eingesetzt wird. Die Pumpe 50 befördert dabei das frische Dialysat vom Behälter 10 zum Konnektor 30. Weiterhin kann die Pumpe 50 das verbrauchte Dialysat vom Konnektor 30 zum Abfluss 20 transportieren.
Ventile 70, welche zur Steuerung der Flüssigkeitsströme eingesetzt werden. Die Ventile 70 öffnen und schließen die Fluidwege 100, um so die entsprechenden Fluidverbindungen zwischen dem Behälter 10, dem Konnektor 30 und dem Abfluss 20 herzustellen.
- Eine Heizung 60, welche das frische Dialysat auf eine Temperatur von ca. 37°C bringt, bevor dieses dem Patienten zugeführt wird. Da bei der Peritonealdialyse relativ große Mengen an Dialysat direkt in die Bauchhöhle des Patienten eingeführt werden, ist die Heizung 60 nötig, um den Patienten nicht zu unterkühlen und um ein unangenehmes Gefühl durch zu kaltes Dialysat zu vermeiden.
- Sensoren 80, über welche der ordnungsgemäße Ablauf der Behandlung überwacht und/oder gesteuert werden kann. Insbesondere können dabei Temperatursensoren zum Einsatz kommen. Weiterhin können gegebenenfalls Drucksensoren zum Einsatz kommen.

Alle Komponenten der Dialysemaschine 40 werden dabei über eine Steuerung 90 angesteuert. Die Steuerung 90 steuert dabei insbesondere die Pumpe 50, die Heizung 60 und die Ventile 70 auf Grundlage der Daten der Sensoren 80 an. Die Steuerung 90 sorgt dabei für den automatischen Ablauf der Peritonealdialyse. Als wichtige Komponente umfasst die Steuerung 90 dabei eine Bilanzierung 95, welche die dem Patienten zugegebenen und entnommenen Flüssigkeitsmengen bilanziert. Die Bilanzierung verhindert dabei, dass dem Patienten zuviel Flüssigkeit zugegeben oder zuviel Flüssigkeit entnommen wird.

Die Bilanzierung 95 kann dabei allein auf Grundlage der Ansteuerdaten und/oder der Sensordaten für die Pumpe 50 erfolgen. Alternativ kann die Bilanzierung auch über separat vorgesehene Bilanzierkammern erfolgen. Ebenso ist es möglich, zur Bilanzierung eine Waage einzusetzen. Eine solche Waage wiegt beispielsweise das Gewicht des Behälters 10 mit frischem Dialysat und/oder eines Behälters 20 mit gebrauchtem Dialysat.

Da bei der Peritonealdialyse das Dialysat dem Patienten direkt in die Bauchhöhle verabreicht wird, ist auf äußerste Sterilität zu achten. Daher werden die Fluidwege bzw. das Fluidsystem, welches mit dem frischen und/oder dem gebrauchten Dialysat in Kontakt kommt, üblicherweise als Einwegteil ausgeführt. Insbesondere werden die Fluidwege bzw. das Fluidsystem dabei als Kunststoffteile ausgeführt. Diese können so in einer sterilen Umverpackung angeliefert und erst kurz vor der Behandlung ausgepackt werden.

Um dennoch eine Steuerung der Peritonealdialyse durch die Dialysemaschine 40 zu ermöglichen, muß das Fluidsystem an die Dialysemaschine 40 angekoppelt werden. In Figur 3 ist dabei schematisch dargestellt, wie einzelne Elemente der Dialysemaschine 40 an entsprechende Bereiche des Fluidsystems angekoppelt werden.

Die Dialysemaschine 40 weist dabei ein Heizelement 61 auf. Dieses muss an einen entsprechenden Heizbereich 62 des Fluidsytems angekoppelt werden. Die Ankopplung ermöglicht dabei die Übertragung von Wärmeenergie von dem Heizelement 61 an das im Heizbereich 62 befindliche Dialysat.

Die Dialysemaschine 40 weist weiterhin einen oder mehrere Pumpaktoren 51 auf, welche mit einem Pumpbereich 52 des Fluidsystems gekoppelt werden. Die Pumpaktoren 51 erzeugen dabei eine Pumpkraft, welche auf den Pumpbereich 52 übertragen wird. Hierdurch kann die im Pumpbereich 52 befindliche Flüssigkeit entlang der Fluidwege bewegt werden.

Weiterhin weist die Dialysemaschine einen oder mehrere Ventilaktoren 71 auf. Diese erzeugen eine Schließbewegung, welche auf entsprechende Ventilbereiche 72 der Fluidwege übertragen wird. Hierdurch können die Ventilbereiche 72 der Fluidwege entsprechend geschlossen bzw. geöffnet werden.

Weiterhin weist die Dialysemaschine einen oder mehrere Sensoren 81 auf. Diese werden an einen entsprechenden Sensorbereiche 82 des Fluidsystems angekoppelt. Hierdurch können die Sensoren 81 gewisse Eigenschaften des Dialysats messen. Insbesondere kann hierdurch die Temperatur des Dialysats gemessen werden. Weiterhin kann vorgesehen sein, dass der Druck in dem Fluidsystem bestimmt wird.

Selbstverständlich weist die Dialysemaschine gegebenenfalls noch weitere Aktoren und/oder Sensoren auf, welche nicht mit den Fluidwegen gekoppelt werden müssen.

Die einzelnen Komponenten eines Peritonealdialysesystems sollen nun im folgenden näher anhand von Ausführungsbeispielen dargestellt werden.

### 1. Fluidsystem

### 1.1 Dialysatbehälter

Frisches Dialysat wird üblicherweise in Kunststoffbeuteln zur Verfügung gestellt. Solche Kunststoffbeutel weisen üblicherweise zwei Lagen von Kunststofffolie auf, welche in einem Randbereich miteinander verschweißt sind und so einen Behälter bilden, welcher mit frischem Dialysat befüllt ist. An diesen Behälter ist üblicherweise ein Schlauchelement angeschweißt, durch welches das Dialysat aus dem Beutel entnommen werden kann. An dem Schlauchelement ist üblicherweise ein Konnektor angeordnet, über welchen der Dialysatbehälter mit den übrigen Fluidwegen verbunden werden kann. Weiterhin weist der Beutel üblicherweise an der dem Schlauch gegenüberliegenden Seite eine Aussparung oder Öse auf, über welche der Beutel an einen Haken aufgehängt werden kann. Hierdurch kann sichergestellt werden, dass das Dialysat problemlos aus dem Beutel abfließt.

Das Dialysat besteht üblicherweise aus einem Puffer, einem Osmotikum und Elektrolyten. Als Puffer kann dabei z. B. Bicarbonat eingesetzt werden. Als Osmotikum wird üblicherweise Glucose eingesetzt. Alternativ können auch Glucosepolymere oder Glucosepolymerderivate eingesetzt werden. Die Elektrolyte umfassen üblicherweise Kalzium und Natrium.

Das Dialysat kann dabei hitzesterilisiert werden. Dies erfolgt vorteilhafterweise, nachdem das Dialysat in den Beutel gefüllt wurde. Hierdurch werden sowohl das

Dialysat als auch der Beutel hitzesterilisiert. Dabei wird der gefüllte Beutel üblicherweise zunächst in eine Umverpackung verpackt, woraufhin das gesamte System sterilisiert wird.

Da die fertige Dialysatlösung je nach Inhaltsstoffen oft nicht hitzesterilisiert werden kann oder nicht lange gelagert werden kann, kann vorgesehen sein, einzelne Komponenten des Dialysats getrennt zu lagern und erst kurz vor der Behandlung zusammenzuführen. Eine erste Einzellösung umfasst dabei üblicherweise den Puffer, während eine zweite Einzellösung Glucose und Elektrolyte umfasst. Gegebenenfalls können auch mehr als zwei Einzellösungen und damit mehr als zwei Bereiche in einem Beutel vorgesehen sein. Dabei kann ein Mehrfachkammerbeutel, insbesondere ein Doppelkammerbeutel vorgesehen sein, welcher mehrere getrennte Bereiche zur Lagerung der Einzellösungen aufweist. Diese Bereiche sind durch ein Verbindungselement getrennt, welches mechanisch geöffnet werden kann, um die Einzelflüssigkeiten miteinander zu vermischen. Insbesondere kann dabei eine sogenannte Peel-Naht zwischen den beiden Bereichen des Beutels vorgesehen sein, welche sich bei Anwendung eines bestimmten Druckes auf mindestens einen der Bereiche des Beutels öffnet.

Da während einer nächtlichen Peritonealdialysebehandlung relativ große Mengen an Dialysat verbraucht werden, werden üblicherweise mehrere Dialysatbehälter parallel eingesetzt. Diese werden über entsprechende Konnektoren mit den Fluidwegen verbunden und können durch eine entsprechende Schaltung der Ventile zum Befüllen des Patienten herangezogen werden.

### 1.2 Abfluss

Zum Entsorgen der verbrauchten Dialysierflüssigkeit kann diese entweder sofort in die Kanalisation abgeführt oder zunächst in einem Abflussbehälter gesammelt werden. Als Abflussbehälter wird dabei üblicherweise ebenfalls ein Beutel eingesetzt. Dieser ist vor Beginn der Behandlung leer und kann so das verbrauchte Dialysat aufnehmen. Der Beutel kann dann nach Beendigung der Behandlung entsprechend entsorgt werden.

### 1.3 Kassette

Wie bereits eingangs beschrieben, weist das Fluidsystem eine Mehrzahl von Bereichen auf, in welchen die Dialysemaschine auf das Fluidsystem einwirken muss. Hierfür muss das Fluidsystem an die Dialysemaschine angekoppelt werden.

Um das Ankoppeln der Fluidwege an die Dialysemaschine und die Einwirkung der entsprechenden Elemente der Dialysemaschine auf die Fluidwege zu vereinfachen, werden Kassetten eingesetzt. In einer solchen Kassette sind mehrere Bereiche, in welchen die Dialysemaschine auf die Fluidwege einwirkt, gemeinsam angeordnet. Hierfür weist eine Kassette üblicherweise ein Hartteil aus Kunststoff auf, in welches zu einer Seite hin offene Kammern als Fluidwege eingebracht sind. Diese Kammern werden von einer flexiblen Kunststofffolie bedeckt, welche für die Ankopplung an die Dialysemaschine sorgt. Die flexible Kunststofffolie ist dabei üblicherweise in einem Randbereich mit dem Hartteil verschweißt. Die Kassette wird mit einer Ankoppelfläche der Dialysemaschine verpresst, so dass die Aktoren und/oder Sensoren der Dialysemaschine mit entsprechenden Bereichen der Kassette in Kontakt kommen.

Die Kassette weist weiterhin Anschlüsse zum Anschluss des Dialysatbehälters 10, des Konnektors 30 sowie des Ablaufs 20 auf.

Eine Kassette umfasst dabei üblicherweise zumindest einen Pumpbereich und einen oder mehrere Ventilbereiche. Über die Kassette kann so der Flüssigkeitstransport durch das Fluidsystem gesteuert werden. Weiterhin kann die Kassette Sensorbereiche aufweisen, welche eine einfache Ankopplung von Sensoren der Dialysemaschine an das Fluidsystem ermöglichen. Gegebenenfalls kann die Kassette weiterhin einen oder mehrere Heizbereiche aufweisen, welche an entsprechende Heizelemente der Dialysemaschine ankoppelbar sind.

In Figuren 4a und 4b ist ein erstes Ausführungsbeispiel einer Kassette dargestellt. Diese weist ein Hartteil 101 aus Kunststoff auf, in welchem die Fluidwege und Ankopplungsbereiche als entsprechende Aussparungen, Kammern und Kanäle eingebracht sind. Das Hartteil kann dabei z. B. als Spritzgussteil oder als Tiefziehteil gefertigt werden. Die Ankoppelebene des Hartteils 101 wird von einer flexiblen Folie 102 bedeckt, welche in einem Randbereich mit dem Hartteil verschweißt ist. Durch das Verpressen der Kassette mit einer Ankoppelfläche der Dialysemaschine wird die flexible Folie 102 mit dem Hartteil verpresst. Durch das Verpressen der flexiblen Folie mit den Stegbereichen des Hartteils werden die Fluidwege innerhalb der Kassette fluiddicht voneinander getrennt.

Die Kassette weist Anschlüsse zum Anschluss der Kassette an die übrigen Fluidwege auf. Zum einen ist ein Anschluss 21 zum Anschluss an den Abfluss 20 sowie ein Anschluss 31 zum Anschluss an den Konnektor 30 vorgesehen. An diesen Anschlüssen können entsprechende Schlauchelemente vorgesehen sein, welche in Figur 4a nicht dargestellt sind. Weiterhin weist die Kassette eine Mehrzahl von Anschlüssen 11 zum Anschluss von Dialysatbehältern 10 auf. Die Anschlüsse 11 sind dabei im ersten Ausführungsbeispiel als Konnektoren ausgeführt, an welche entsprechende Konnektorelemente angeschlossen werden können.

Die Anschlüsse stehen jeweils mit Fluidwegen innerhalb der Kassette in Verbindung. In diesen Fluidwegen sind Ventilbereiche vorgesehen. In diesen Ventilbereichen kann die flexible Folie 102 über maschinenseitige Ventilaktoren so in das Hartteil 101 gedrückt werden, dass der entsprechende Fluidweg versperrt ist. Die Kassette weist dabei zunächst einmal für jeden Anschluss ein entsprechendes Ventil auf, über welches dieser Anschluss geöffnet bzw. geschlossen werden kann. Dem Anschluss 21 für den Abfluss 20 ist dabei das Ventil V10 zugeordnet, dem Anschluss 31 für den Patientenkonnektor 30 das Ventil V6. Den Anschlüssen 11 für die Dialysatbehälter 10 sind die Ventile V11 bis V16 zugeordnet.

Weiterhin sind in der Kassette Pumpenkammern 53 und 53' vorgesehen, welche durch entsprechende Pumpaktoren der Dialysemaschine betätigt werden können. Bei den Pumpenkammern 53 und 53' handelt es sich dabei um konkave Aussparungen in dem Hartteil 101, welche von der flexiblen Folie 102 bedeckt werden. Durch Pumpaktoren der Dialysemaschine kann die Folie nun in die Pumpenkammern 53 und 53' hineingedrückt bzw. wieder aus diesen Pumpenkammern herausgezogen werden. Hierdurch kann, im Zusammenspiel mit den Ventilen V1 bis V4, welche die Zugänge und Abläufe der Pumpenkammern 53 und 53' schalten und in Fig. 4a mit dem Bezugszeichen 73 bezeichnet wurden, ein Pumpstrom durch die Kassette erzeugt werden. Die Pumpkammern sind dabei über entsprechende Ventilschaltungen mit allen Anschlüssen der Kassette verbindbar.

Weiterhin ist ein Heizbereich 62 in die Kassette integriert. In diesem Bereich wird die Kassette mit Heizelementen der Dialysemaschine in Kontakt gebracht, welche das durch diesen Bereich der Kassette fließende Dialysat erwärmen. Der Heizbereich 62 weist dabei einen Kanal für das Dialysat auf, welcher sich spiralförmig über den Heizbereich 62 erstreckt. Der Kanal wird dabei durch Stege 64 des Hartteils gebildet, welche von der flexiblen Folie 102 abgedeckt sind.

Der Heizbereich 62 ist dabei auf beiden Seiten der Kassette vorgesehen. Hierfür ist auch auf der Unterseite 63 der Kassette im Heizbereich eine flexible Folie am Hartteil angeordnet. Die flexible Folie ist dabei ebenfalls in einem Randbereich mit dem Hartteil verschweißt. Auf der Unterseite ist ebenfalls ein Kanal angeordnet, durch welchen das Dialysat fließt. Die Kanäle auf der Unterseite und der Oberseite werden dabei durch eine mittlere Platte des Hartteils gebildet, welche die Oberseite von der Unterseite trennt, und auf welcher nach unten und nach oben Stege vorgesehen sind, welche die Kanalwandungen bilden. Dabei fließt das Dialysat zunächst spiralförmig auf der Oberseite bis zum Durchbruch 65 durch die mittlere Platte, von wo aus das Dialysat auf der Unterseite durch den entsprechenden Kanal zurückfließt. Durch den auf der Ober- und der Unterseite vorgesehenen Heizbereich kann die Heizfläche, welche zum Aufheizen der Flüssigkeit zur Verfügung steht, entsprechend vergrößert werden. Selbstverständlich ist aber auch eine Ausführungsform der Kassette möglich, bei welcher nur auf einer Seite der Kassette ein Heizbereich angeordnet ist.

Weiterhin sind Ausführungsformen der Kassette möglich, bei welcher ein Heizelement in die Kassette integriert ist. Insbesondere kann dabei ein elektrisches Heizelement wie z.B. Heizwendel in das Hartteil der Kassette eingegossen sein. Hierdurch kann auf ein maschienenseitiges Heizelement verzichtet werden und die Durchflussheizung in die Kassette integriert werden. Dabei sind an der Kassette elektrische Kontakte zur Konnektierung des elektrischen Heizelements angeordnet.

Die Kassette weist weiterhin Sensorbereiche 83 und 84 auf, durch welche Temperatursensoren der Dialysemaschine an die Kassette gekoppelt werden können. Die Temperatursensoren liegen dabei auf der flexiblen Folie 102 auf und können so die Temperatur der durch den darunter liegenden Kanal fließenden Flüssigkeit messen. Dabei sind am Eingang des Heizbereiches zwei Temperatursensoren 84 vorgesehen. Am patientenseitigen Ausgang ist ein Temperatursensor 83 vorgesehen, über welchen die Temperatur des zum Patienten gepumpten Dialysats gemessen werden kann.

In Figur 5 ist ein zweites Ausführungsbeispiel für eine Kassette gezeigt. Die Kassette entspricht dabei in ihrer Ausführung im wesentlichen dem ersten Ausführungsbeispiel, umfasst jedoch keinen Heizbereich. Bei Einsatz dieser Kassette erfolgt die Heizung daher nicht wie beim ersten Ausführungsbeispiel gezeigt über einen in die Kassette integrierten Heizbereich, sondern z.B. über einen Heizbeutel, welcher auf eine Heizplatte der Dialysemaschine gelegt wird.

Das in Figur 5 gezeigte zweite Ausführungsbeispiel einer Kassette weist wiederum Fluidwege auf, welche über Ventilbereiche, die hier ebenfalls von V1 bis V16 durchnumeriert sind, geöffnet und geschlossen werden können. Weiterhin weist die Kassette Anschlüsse zum Anschluss an weitere Komponenten des Fluidsystems auf. Dabei ist wiederum der Anschluss 21 zum Anschluss an den Abfluss 20, sowie der Anschluss 31 zum Anschluss an den Konnektor 30 zum Patienten vorgesehen. Weiterhin sind Anschlüsse 11 zum Anschluss von Dialysatbehältern 10 vorgesehen.

Im Unterschied zum ersten Ausführungsbeispiel weist die im zweiten Ausführungsbeispiel gezeigte Kassette einen weiteren Anschluss 66 zum Anschluss eines Heizbeutels auf. Zum Erwärmen der Flüssigkeit aus den Dialysatbehältern 10 kann die Flüssigkeit dabei über den Anschluss 66 in einen Heizbeutel gepumpt werden. Dieser Heizbeutel liegt auf einem Heizelement auf, so dass die im Heizbeutel befindliche Flüssigkeit erwärmt werden kann. Daraufhin wird die Flüssigkeit aus dem Heizbeutel zum Patienten gepumpt.

Die Pumpkammer 53 und 53' und die Ventile V1 bis V4 entsprechen in Aufbau und Funktion den entsprechenden Komponenten im ersten Ausführungsbeispiel.

Im Unterschied zum ersten Ausführungsbeispiel weist die Kassette im zweiten Ausführungsbeispiel keinen Sensorbereich zum Anschluss eines Temperatursensors auf. Dieser ist vielmehr im Bereich der Heizelemente angeordnet. Die Kassette weist jedoch Messbereiche 85 und 86 zum Messen des Drucks in den Pumpekammern 53 und 53' auf. Die Messbereiche 85 und 86 sind dabei Kammern, welche mit dem Pumpkammern fluidisch in Verbindung stehen und ebenfalls von der flexiblen Folie abgedeckt werden. An die Messbereiche können geräteseitige Drucksensoren angekoppelt werden, welche den Druck in den Messkammern 85 und 86 und damit in den Pumpkammern 53 und 53' messen.

Die Verbindung der Anschlüsse 11, 21, 31 und 66 der Kassette mit den weiteren Komponenten des Fluidsystems erfolgt im zweiten Ausführungsbeispiel über Schlauchverbindungen. An diesen Schlauchverbindungen sind gegebenenfalls Konnektoren angeordnet.

### 1.3 Schläuche

Die Verbindung zwischen den einzelnen Behältern des Systems, der Kassette und dem Patientenkonnektor erfolgt üblicherweise über Schlauchverbindungen. Da es sich jeweils um Einwegartikel handelt, sind die Schläuche dabei üblicherweise zumindest auf einer Seite bereits mit einem weiteren Element fest verbunden. Z. B. können Schläuche bereits an einem oder mehreren der Anschlüsse der Kassette vorgesehen sein. Ebenfalls können Schläuche bereits fest mit Beuteln in Verbindung stehen.

### 1.4 Verbindungen

Das Fluidsystem ist üblicherweise in mehrere Teile aufgeteilt und jeweils steril verpackt. Diese Teile müssen für die Behandlung zunächst miteinander verbunden werden. Insbesondere sind dabei üblicherweise die Kassette sowie der oder die Dialysatbeutel getrennt voneinander verpackt.

Die Verbindungen zwischen den einzelnen Elementen des Fluidsystems erfolgt üblicherweise über Konnektoren. Die Konnektoren sind dabei so gestaltet, dass sie eine sterile Verbindung zwischen den einzelnen Komponenten ermöglichen. Dies erfolgt z. B. über entsprechende Schutzfolien, welche beim Schließen des Konnektors automatisch geöffnet werden.

Die Verbindung der einzelnen Komponenten kann dabei manuell durch eine Bedienperson bzw. den Patienten selbst erfolgen. Alternativ kann vorgesehen sein, dass die Verbindung der einzelnen Komponenten durch die Dialysemaschine erfolgt.

Hierzu können z. B. die entsprechenden Konnektoren in eine Konnektoraufnahme der Dialysemaschine eingelegt und automatisch durch die Dialysemaschine zusammengeführt werden.

Weiterhin kann eine elektronische Steuerung vorgesehen sein, welche überwacht, dass die richtigen Komponenten des Systems miteinander verbunden werden. Hierfür können an den Konnektoren Identifikationsmittel wie z. B. Barcodes oder RFIDs vorgesehen sein, welche die Komponenten identifizieren. Die Dialysemaschine umfasst dabei eine Identifikationsmittel-Erfassungseinheit wie z. B. einen Barcodeleser oder eine RFID-Erfassungseinheit, welcher die Identifikationsmittel auf den Konnektoren erfasst. Hierdurch kann die Steuerung der Peritonealdialyse erkennen, ob die korrekten Konnektoren eingelegt wurden.

Eine solche Überprüfung der korrekten Zusammenstellung des Fluidsystems kann dabei insbesondere mit einer automatischen Konnektion der Konnektoren kombiniert sein. Das System überprüft so zunächst, ob die richtigen Konnektoren in die Konnektorenaufnahmen eingelegt wurden. Die Verbindung zwischen den Konnektoren wird durch die Dialysemaschine nur hergestellt, wenn die richtigen Konnektoren eingelegt wurden. Andernfalls macht die Dialysemaschine den Benutzer darauf aufmerksam, dass die falschen Konnektoren eingelegt wurden.

### 2. Die Dialysemaschine

Die einzelnen Komponenten einer Dialysemaschine sollen nun im folgenden anhand von zwei Ausführungsbeispielen näher beschrieben werden.

In Figur 6 ist dabei ein erstes Ausführungsbeispiel einer Dialysemaschine gezeigt, bei welchem das erste Ausführungsbeispiel einer Kassette eingesetzt wird. Das sich aus dem ersten Ausführungsbeispiel einer Dialysemaschine und dem ersten Ausführungsbeispiel einer Kassette ergebende Peritonealdialysesystem ist dabei in Figur 7 gezeigt.

In Figur 8 ist ein zweites Ausführungsbeispiel einer Dialysemaschine gezeigt, bei welcher das zweite Ausführungsbeispiel einer Kassette zum Einsatz kommt. Das sich aus der Kombination des zweiten Ausführungsbeispiels einer Dialysemaschine und des zweiten Ausführungsbeispiels einer Kassette ergebende Dialysesystem ist dann in Figur 9 gezeigt.

Die beiden Ausführungsbeispiele unterscheiden sich dabei zum einen in der Ausgestaltung der Heizung, in der Kopplung zwischen der Dialysemaschine und der Kassette sowie in der Ausgestaltung der Aktoren und Sensoren.

### 2.1 Heizung

Das frische Dialysat muss auf Körpertemperatur gebracht werden, bevor es in den Bauchraum des Patienten befördert wird. Hierfür weist die Dialysemaschine eine entsprechende Heizung auf.

Die Heizung erfolgt dabei üblicherweise elektrisch über ein oder mehrere Heizelemente. Bei den Heizelementen kann es sich dabei z. B. um keramische Heizelemente handeln. Bei solchen keramischen Heizelementen ist eine Widerstandsbahn auf einem Keramikträger aufgebracht. Durch Anlegen einer Spannung an die Widerstandsbahn wird diese erhitzt, wodurch sich auch das keramische Trägermaterial erhitzt. Das keramische Heizelement ist dabei üblicherweise auf einer Heizplatte angeordnet. Diese kann beispielsweise aus Aluminium gefertigt sein. An die Heizplatte werden wiederum die Fluidwege angekoppelt, so dass das in den Fluidwegen befindliche Dialysat erhitzt werden kann.

Zum Erwärmen der Flüssigkeit stehen zwei unterschiedliche Ausgestaltungen zur Verfügung. Zum einen kann zunächst eine größere Menge an Dialysat erwärmt werden, welche erst nach der Aufwärmphase zum Patienten gepumpt wird. Dies erfolgt üblicherweise über einen Heizbeutel, welcher auf einer Heizplatte des Dialysegerätes aufliegt.

Bei dem Heizbeutel kann es sich dabei um den Dialysatbeutel handeln, in welchem das Dialysat zur Verfügung gestellt wird. Üblicherweise wird jedoch ein separater Heizbeutel eingesetzt, in welchem das Dialysat zum Aufheizen hineingepumpt wird. Ist das Dialysat im Heizbeutel erwärmt, wird es von dort aus zum Patienten gepumpt.

Ein solches Konzept ist bei den in Figuren 8 und 9 gezeigten zweiten Ausführungsbeispiel einer Dialysemaschine verwirklicht. Dabei ist ein Heizbeutel 67 vorgesehen, welcher auf einer Heizplatte 68 aufliegt. Die Heizplatte 68 ist dabei auf der Oberseite des Peritonealdialysegerätes angeordnet, so dass sie leicht zugänglich ist. Der Heizbeutel 67 ist dabei über eine Leitung 66' mit der Kassette verbunden. Die Kassette weist dabei die Ventile V5, V9 und V15 auf, über welche der Heizbeutel 67 mit den übrigen Komponenten des Fluidsystems verbunden werden kann. So kann frisches Dialysat von den Dialysatbehältern 10 über die Pumpkammern zum Heizbeutel 67 gepumpt werden. Zu Beginn einer Behandlung wird also zunächst der Heizbeutel 67 mit kaltem Dialysat gefüllt. Das Dialysat im Heizbeutel 67 wird dann über die Heizplatte 68 auf Körpertemperatur erwärmt. Daraufhin wird das Dialysat über die Pumpkammern zum Patienten gepumpt. Daraufhin kann der Heizbeutel 67 wieder befüllt werden, so dass die für den nächsten Behandlungszyklus benötigte Dialysatmenge erhitzt werden kann.

Vorteilhafterweise ist dabei im Bereich der Heizplatte 68 ein Temperatursensor 88 vorgesehen, welcher mit dem Heizbeutel 67 in Kontakt steht und so die Temperatur des Dialysats im Heizbeutel 67 messen kann. Weiterhin kann ein Temperatursensor an der Heizplatte oder am Heizelement vorgesehen sein, welcher die Temperatur des Heizelements oder der Heizplatte misst. Eine entsprechende Steuerung sorgt nun dafür, dass die Heizplatte nicht zu heiß für das Material des Beutels wird.

Der Heizbeutel 67 kann zudem Funktionen bei der Billanzierung der Flüssigkeitsströme übernehmen. So kann die Heizplatte 68 Teil einer Waage 87 sein, über welche das Gewicht des Heizbeutels 67 bestimmbar ist. Hierdurch kann die Flüssigkeitsmenge, welche nach dem Erwärmen dem Patienten zugeführt wird, bestimmt werden.

Alternativ zu der beim zweiten Ausführungsbeispiel gezeigten Erwärmung des Dialysats über einen Heizbeutel kann das Dialysat auch erwärmt werden, während es zum Patienten gepumpt wird. Die Heizung arbeitet damit in Form eines Durchlauferhitzers, welcher das durch das Fluidsystem bewegte Dialysat erwärmt, während es durch die Fluidwege gepumpt wird.

Bei diesem Konzept ist ein Dialysatkanal vorgesehen, welcher an ein Heizelement der Dialysemaschine gekoppelt wird. Während das Dialysat durch den Dialysatkanal fließt, nimmt es dabei Wärme von dem Heizelement der Dialysemaschine auf.

Ein solches Konzept ist bei dem ersten Ausführungsbeispiel einer Dialysemaschine, welches in Figuren 6 und 7 gezeigt ist, implementiert. Dabei ist der Heizbereich in die Kassette integriert, wie dies bereits oben dargestellt wurde. Beim Ankoppeln der Kassette an die Dialysemaschine kommt dabei der Heizbereich der Kassette mit Heizelementen der Dialysemaschine thermisch in Kontakt.

Die Heizelemente können dabei ebenfalls als keramische Heizelemente ausgeführt sein und mit Heizplatten in Kontakt stehen, welche dann an den Heizbereich der Kassette gekoppelt werden. Wie bereits hinsichtlich der Kassette dargestellt, steht dabei sowohl mit der Oberseite als auch mit der Unterseite des Heizbereichs jeweils eine Heizplatte in Kontakt, welche das durch den Heizbereich fließende Dialysat erwärmt.

Am Zulauf und am Ablauf des Heizbereichs sind jeweils Temperatursensorbereiche in der Kassette vorgesehen, welche durch das Ankoppeln der Kassette mit Temperatursensoren der Peritonealdialyse in Kontakt kommen. Durch die Temperatursensoren T1 bis T3 kann so die Temperatur des in den Heizbereich einfließenden Dialysats sowie die Temperatur des aus dem Heizbereich herausfließenden Dialysats bestimmt werden. Weiterhin sind Temperatursensoren T4 und T5 vorgesehen, welche die Temperatur der Heizelemente und/oder der Heizplatten bestimmen.

Die Verwendung von mindestens zwei Heizelementen ermöglicht es dabei, die Heizelemente jeweils so zusammenzuschalten, dass sie bei einer Versorgungsspannung von 220 V im wesentlichen die gleiche Leistung abgeben wird wie bei einer Versorgungsspannung von 110 V. Hierfür werden die beiden Heizelemente bei 110 V in einer Parallelschaltung betrieben, während sie bei einer Versorgungsspannung von 220 V in einer Seriellschaltung betrieben werden. Eine solche Anpassung der Verschaltung der Heizelemente an die Versorgungsspannung kann dabei unabhängig davon, ob die Heizung gemäß dem ersten oder dem zweiten Ausführungsbeispiel erfolgt, implementiert werden.

### 2.2 Ankopplung der Kassette

Um eine Ankopplung der Aktoren und/oder Sensoren der Dialysemaschine an die entsprechenden Bereiche der Kassette zu ermöglichen, weist die Dialysemaschine eine Kassettenaufnahme mit einer Ankoppelfläche auf, an welcher die Kassette angekoppelt werden kann. An der Ankoppelfläche sind die entsprechenden Aktoren, Sensoren und/oder Heizelemente der Dialysemaschine angeordnet. Die Kassette wird mit dieser Ankoppelfläche so verpresst, dass die entsprechenden Aktoren, Sensoren und/oder Heizelemente mit den entsprechenden Bereichen an der Kassette in Kontakt kommen.

Dabei ist vorteilhafterweise an der Ankoppelfläche der Dialysemaschine eine Matte aus einem flexiblen Material vorgesehen, insbesondere eine Silikonmatte. Diese sorgt dafür, dass die flexible Folie der Kassette mit den Stegbereichen der Kassette verpresst wird und so die Fluidwege innerhalb der Kassette voneinander trennt.

Vorteilhafterweise ist weiterhin ein umlaufender Rand der Ankoppelfläche vorgesehen, welcher mit dem Randbereich der Kassette verpresst wird. Vorteilhafterweise erfolgt die Verpressung dabei luftdicht, so dass zwischen der Ankoppelfläche und der Kassette ein Unterdruck aufgebaut werden kann.

Gegebenenfalls kann auch ein Vakuumsystem vorgesehen sein, welches Luft aus dem Raum zwischen Ankoppelfläche und Kassette abpumpen kann. Hierdurch wird eine besonders gute Ankopplung der Aktoren, Sensoren und/oder Heizelemente des Peritonealdialysegerätes mit den entsprechenden Bereichen der Kassette ermöglicht. Zudem erlaubt das Vakuumsystem eine Dichtigkeitsprüfung der Kassette. Hierfür wird nach dem Ankoppeln ein entsprechendes Vakuum angelegt und überprüft, ob dieses Vakuum aufrechterhalten wird.

Das Anpressen der Kassette erfolgt z. B. pneumatisch. Hierzu ist üblicherweise ein Luftkissen vorgesehen, welches mit Druckluft befüllt wird und so die Kassette an die Ankoppelfläche anpresst.

Die Kassettenaufnahme weist üblicherweise eine der Ankoppelfläche gegenüberliegende Aufnahmefläche auf, in welche das Hartteil der Kassette eingelegt wird. Die Aufnahmefläche weist hierfür vorteilhafterweise entsprechende Vertiefungen auf. Die Aufnahmefläche mit der eingelegten Kassette kann dann über eine pneumatische Anpressvorrichtung an die Ankoppelfläche angepresst werden.

Das Einlegen der Kassette kann dabei in unterschiedlicher Weise geschehen. Im ersten Ausführungsbeispiel einer Dialysemaschine, welches in Figur 6 gezeigt ist, ist hierfür eine Schublade 111 vorgesehen, welche aus der Dialysemaschine ausgefahren werden kann. In diese Schublade wird die Kassette eingelegt. Die Kassette wird dann zusammen mit der Schublade in die Dialysemaschine eingeschoben. Daraufhin erfolgt das Verpressen der Kassette mit der Ankoppelfläche, welche im Inneren des Gerätes angeordnet ist. Dabei werden Kassette und Koppelfläche zunächst mechanisch aneinander gefahren und dann pneumatisch miteinander verpresst.

Das Ankoppeln einer Kassette 110 gemäß dem zweiten Ausführungsbeispiel ist in Figur 10 näher dargestellt. Die Ankoppelfläche 130 ist durch Öffnen einer Tür 140 frei zugänglich, so dass die Kassette in der richtigen Position an der Ankoppelfläche 130 angeordnet werden kann. Die Ankoppelfläche 130 ist dabei nach hinten zur Vertikalen geneigt, was ein leichteres Ankoppeln ermöglicht. Nun kann die Tür 140 geschlossen werden, so dass eine Aufnahmefläche an der Tür mit der Rückseite der Kassette in Kontakt kommt. Das Verpressen erfolgt nun durch ein an der Tür angeordnetes Luftkissen. Zudem wird ein Vakuum zwischen die Ankoppelfläche und die Kassette 110 angelegt.

Das erste Ausführungsbeispiel einer Dialysemaschine weist weiterhin eine Vorrichtung zum automatischen Konnektieren auf. Hierfür ist eine Konnektorenaufnahme 112 vorgesehen, in welche die Konnektoren der Dialysatbeutel 10 eingelegt werden. Die Konnektorenaufnahme 112 fährt dann in das Gerät hinein, wo ein Barcodeleser vorgesehen ist, welcher die auf den Konnektoren aufgebrachten Barcodes liest. So kann das Gerät überprüfen, ob die richtigen Beutel eingelegt wurden. Werden die richtigen Beutel erkannt, so fährt die Konnektoraufnahme 112 komplett ein und schließt so die Konnektoren der Beutel an den als Konnektoren ausgeführten Anschlüssen 11 der Kassette an.

Im zweiten Ausführungsbeispiel wurde auf eine solche automatische Konnektierung dagegen verzichtet. Daher sind an den Anschlüssen 11 der Kassette Schlauchabschnitte angeordnet, welche manuell über Konnektoren mit den entsprechenden Beuteln verbunden werden müssen.

### 2.3 Pumpaktoren

Das Pumpen der Flüssigkeit durch das Fluidsystem erfolgt in den Ausführungsbeispielen durch eine Membranpumpe, welche von den Pumpkammern 53 und 53' zusammen mit der flexiblen Folie der Kassette gebildet wird. Wird die flexible Folie dabei durch einen entsprechenden Pumpaktor in die Pumpkammer hineingedrückt, so wird Flüssigkeit aus der Pumpkammer in die geöffneten Bereiche der Fluidwege der Kassette gepumpt. Umgekehrt wird durch Herausziehen der Folie aus der Pumpkammer Fluid aus den Fluidwegen in die Pumpkammer gesaugt.

Der Pumphub erfolgt dabei durch Bewegen eines Pumpaktors in die Pumpkammer. Für den Saughub wird der Pumpaktor wieder von der Pumpkammer wegbewegt. Durch die luftdichte Verpressung von Kassette und Ankoppelfläche entsteht dabei ein Unterdruck, durch welchen die flexible Folie der Kassette dem Pumpaktor folgt und so wieder aus der Pumpkammer herausgezogen wird.

Um eine gute Ankopplung des Pumpaktors an die flexible Folie der Kassette zu ermöglichen, kann zudem ein Vakuumsystem vorgesehen sein. Über das Einstellen eines entsprechenden Vakuums zwischen der Ankoppelfläche und der Kassette kann dabei insbesondere die Kraft eingestellt werden, mit welcher die flexible Folie während eines Saughubs maximal von der Pumpkammer wegbewegt wird.

Hierdurch kann die Saugkraft der Pumpe sehr fein eingestellt werden. Die Pumpkraft wird dagegen durch die Schubkraft des Aktors eingestellt.

Die Bilanzierung der Flüssigkeitsströme kann dabei durch das Zählen der Saug- und Pumphübe erfolgen, da die Membranpumpe eine hohe Genauigkeit der mit jedem Hub gepumpten Flüssigkeitsmenge aufweist.

### 2.3.1. Hydraulischer Antrieb

Der Aufbau eines ersten Ausführungsbeispiels eines Pumpaktors ist in Figur 11 gezeigt. Der Pumpaktor wird dabei hydraulisch bewegt. Hierfür ist eine Membran 59 vorgesehen, welche an der flexiblen Folie der Kassette anlegt. Die Membran 59 kann dabei z. B. aus Silikon gefertigt sein. Hinter der Membran 59 ist eine Kammer 54 vorgesehen, welche mit Hydraulikfluid gefüllt werden kann. Durch Anlegen eines Überdrucks in der Kammer 54 wird die Membran 59 und mit dieser die flexible Folie in die Pumpkammer 53 der Kassette hineingedrückt. Durch Anlegen eines Unterdruckes an die Kammer 54 wird die Membran 59 dagegen in die Kammer 54 hineingezogen. Durch den Unterdruck zwischen der flexiblen Folie und der Membran folgt die flexible Folie dieser Bewegung, so dass sich das Volumen der Pumpkammer 53 vergrößert. Der Pumpvorgang mit den Pumphub und dem Ansaughub ist dabei schematisch in Figur 12b dargestellt.

Zum Betrieb der Pumphydraulik ist eine Hydraulikpumpe 58 vorgesehen. Diese weist einen Zylinder auf, in welchem ein Kolben über einen Motor 57 hin und her bewegt werden kann. Hierdurch wird die Hydraulikflüssigkeit über eine entsprechende Verbindungsleitung in die Kammer 54 hineingepreßt oder aus dieser wieder herausgesogen. An der Hydraulikpumpe 58 ist dabei ein Wegaufnehmer 56 vorgesehen, über welchen die Bewegung des Kolbens aufgenommen werden kann. Hierdurch kann bestimmt werden, wieviel Hydraulikflüssigkeit in die Kammer 54 hineingepreßt bzw. wieviel Hydraulikflüssigkeit aus dieser entnommen wurde. Weiterhin sind Drucksensoren 55 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 56 verglichen werden können und hierdurch die Dichtigkeit des Hydrauliksystems überprüft werden kann.

Zudem ermöglichen die Drucksensoren eine Bestimmung des Drucks in der Pumpekammer 53 der Kassette. Wird die Hydraulikpumpe 58 nicht bewegt, so stellt sich ein Druckgleichgewicht zwischen der Kammer 54 und der Pumpkammer 53 ein. Der Druck der Hydraulikflüssigkeit entspricht damit dem Druck in der Pumpenkammer 53.

In Figur 12a ist nun der Ankoppelvorgang des Pumpenaktors an die Pumpenkammer 53 gezeigt. Zur Vorbereitung des Ankoppelns wird dabei zunächst die Kammer 54 mit Hydraulikfluid beaufschlagt, dass sich die Membran 59 nach außen wölbt. Daraufhin werden Ankoppelfläche und Kassette aufeinander zu bewegt, so dass die Membran 59 die flexible Folie der Kassette in die Pumpkammer 53 hineindrückt. Nach dem Verpressen von Ankoppelfläche und der Kassette ist der Raum zwischen der Membran und der flexiblen Folie nach außen luftdicht abgeschlossen, so dass die flexible Folie der Bewegung der Membran folgt. Dies ist in Figur 12b gezeigt.

Der in Figur 11 gezeigte Pumpaktor ist dabei beim ersten Ausführungsbeispiel einer Dialysemaschine implementiert, wie dies auch aus Figur 7 ersichtlich ist. Dabei ist für jede der beiden Pumpkammern 53 und 53' jeweils ein entsprechender Pumpaktor vorgesehen.

### 2.3.2 Elektromechanischer Antrieb

Alternativ kann der Pumpaktor auch elektromotorisch betrieben werden. Hierfür ist ein entsprechend geformter Stempel vorgesehen, welcher über einen Elektromotor, insbesondere über einen Schrittmotor gegen die flexible Folie gedrückt bzw. von dieser wegbewegt wird und so den Pump- bzw. Saughub erzeugt. Solche Pumpaktoren 151 und 152 sind bei dem Ausführungsbeispiel in Fig. 10 gezeigt. Vorteilhafterweise ist dabei ein Vakuumsystem vorgesehen, welches dafür sorgt, dass die flexible Folie dem Stempel auch bei der Saugbewegung folgt.

### 2.4 Ventilaktoren

Als Ventilaktor kann ein Ventilstößel vorgesehen sein, welcher die flexible Folie der Kassette in eine entsprechende Kammer des Hartteils hineindrückt und so den Fluidkanal in diesem Bereich schließt. Der Ventilaktor kann dabei Z.B. pneumatisch betätigt werden. Der Stößel kann dabei über eine Feder vorgespannt sein, so dass er entweder drucklos öffnet oder drucklos schließt.

Alternativ kann der Ventilaktor über eine flexible Membran implementiert werden, welche hydraulisch oder pneumatisch bewegt wird. Die flexible Membran wird dabei durch Anlegen von Druck gegen die Kassette bewegt und drückt so einen entsprechenden Ventilbereich der flexiblen Folie in einen Fluidkanal, um diesen zu verschließen.

Ventilaktoren 71, welche an die Ventilbereiche V1 bis V16 der Kassette angekoppelt werden, sind in Fig. 10 auf der Ankoppelfläche erkennbar.

### 2.5 Sensoren

Die Dialysemaschine weist Sensoren auf, über welche die Maschine angesteuert bzw. deren ordnungsgemäßes Funktionieren überwacht werden kann.

Zum einen sind dabei ein oder mehrere Temperatursensoren vorgesehen, über welche die Temperatur des Dialysats und/oder der Heizelemente gemessen werden kann. Im ersten Ausführungsbeispiel sind die Temperatursensoren dabei an der Ankoppelfläche zur Kassette angeordnet und können so die Temperatur des durch die Kassette fließenden Dialysats messen. Im zweiten Ausführungsbeispiel ist dagegen ein Temperatursensor 88 auf der Heizplatte 68 vorgesehen, welcher die Temperatur des im Beutel 67 befindlichen Dialysats mißt. Weiterhin können an dem oder den Heizelementen Temperatursensoren vorgesehen sein.

Weiterhin können ein oder mehrere Drucksensoren vorgesehen sein, um den Druck in den Pumpkammern zu bestimmen. Hierdurch kann verhindert werden, das Dialysat mit zu hohem Druck zum Patienten gepumpt wird oder der Saugdruck beim Absaugen von Dialysat vom Patienten zu hoch wird.

Im ersten Ausführungsbeispiel erfolgt die Druckmessung dabei über Drucksensoren in der Hydraulik der Pumpaktoren, wie dies oben dargestellt wurde. Im zweiten Ausführungsbeispiel sind dagegen Drucksensoren 85' und 86' in der Ankoppelfläche vorgesehen, welche den Druck in entsprechenden Druckmessbereichen der Kassette direkt messen. Die Ankopplung dieser Drucksensoren an die Kassette wird dabei vorteilhafterweise durch ein Vakuumsystem sichergestellt.

### 2.6 Ein-/Ausgabeeinheit

Die Dialysemaschine umfasst weiterhin eine Ein-/Ausgabeeinheit zur Kommunikation mit einem Bediener. Zur Ausgabe von Informationen ist dabei eine entsprechende Anzeige vorgesehen, welche z. B. durch Leuchtdioden, LCD-Anzeigen oder einen Bildschirm implementiert sein kann. Zur Eingabe von Befehlen sind entsprechende Eingabeelemente vorgesehen. Hierbei können z. B. Drucktasten und Schalter vorgesehen sein.

In beiden Ausführungsbeispielen ist dabei ein Touchscreen 120 vorgesehen, welcher eine interaktive Menüführung ermöglicht. Weiterhin sind Anzeigeelemente 121 und 122 vorgesehen, welche Zustände der Dialysemaschine kompakt darstellen.

Das erste Ausführungsbeispiel weist weiterhin einen Kartenleser 125 auf, über welchen eine Patientenkarte eingelesen werden kann. Auf der Patientenkarte können Daten zur Behandlung des jeweiligen Patienten abgespeichert sein. Hierdurch kann der Behandlungsablauf für den jeweiligen Patienten individuell festgelegt werden.

Die Peritonealdialyse weist weiterhin eine akustische Signaleinheit auf, über welche akustische Signale abgegeben werden können. Insbesondere kann dabei ein akustisches Warnsignal ausgegeben werden, wenn ein Fehlzustand registriert wird. Dabei ist vorteilhafterweise ein Lautsprecher vorgesehen, über welchen die akustischen Signale erzeugt werden können.

### 2.7 Steuerung

Die Peritonealdialyse weist weiterhin eine Steuerung auf, durch welche sämtliche Komponenten angesteuert und überwacht werden. Die Steuerung sorgt dabei für den automatischen Ablauf der Behandlung.

In Figur 13 ist nun der prinzipielle Aufbau eines Ausführungsbeispiels einer solchen Steuerung dargestellt.

Die Kommunikation mit dem Bediener sowie mit externen Informationsquellen erfolgt dabei über einen Interface-Rechner 150. Dieser kommuniziert mit einem Patientenkartenleser 200, einer Ein- und Ausgabeeinheit 210, welche der Kommunikation mit dem Patienten dient, sowie mit einem Modem 220. Über das Modem 220 kann z. B. eine aktualisierte Software aufgespielt werden.

Der Interface-Rechner 150 steht über einen internen Bus mit einem Aktionsrechner 160 und einem Schutzrechner 170 in Verbindung. Der Aktionsrechner 160 und der Schutzrechner 170 erzeugen eine Redundanz des Systems. Der Aktionsrechner 160 erhält dabei Signale von den Sensoren des Systems und berechnet die Steuersignale für die Aktoren 180. Der Schutzrechner 170 erhält ebenfalls Signale von den Sensoren 180 und überprüft, ob die vom Aktionsrechner 160 ausgegebenen Befehle korrekt sind. Stellt der Schutzrechner 170 einen Fehler fest, so leitet er eine entsprechende Notfallprozedur ein. Insbesondere kann der Schutzrechner 170 dabei ein Alarmsignal auslösen. Weiterhin kann der Schutzrechner 170 den Zugang zum Patienten schließen. Hierfür ist ein spezielles Ventil am patientenseitigen Ausgang der Kassette angeordnet, auf welches nur der Schutzrechner 170 Zugriff hat. Dieses Sicherheitsventil ist dabei im drucklosen Zustand geschlossen, so dass es bei einem Ausfall der Pneumatik automatisch schließt.

Der Schutzrechner 170 steht weiterhin mit dem Barcodeleser 190 in Verbindung, und überprüft so den Anschluss der korrekten Dialysatbeutel.

Weiterhin ist ein Diagnosesystem 230 vorgesehen, über welches Fehler des Systems ermittelt und behoben werden können.

### 3. Implementierung der Erfindung

Ausführungsbeispiele der beiden Aspekte der vorliegenden Erfindung, welche bei einem oben dargestellten Dialysesystem bzw. bei einer der oben dargestellten Dialysemaschinen zum Einsatz kommen können, werden nun im folgenden dargestellt. Dabei können die Ausführungsbeispiele der vorliegenden Erfindung mit einzelnen oder mehreren Komponenten, wie sie oben beschrieben wurden, kombiniert werden.

In Figur 14a ist eine Prinzipdarstellung eines erfindungsgemäßen Gerätes 300 mit einem Isolationswächter 350 gezeigt. Das Gerät ist dabei über einen Netzspannungsanschluss 310 elektrisch betreibbar. Im Ausführungsbeispiel weist das erfindungsgemäße Gerät dabei keinen Schutzleiteranschluss auf. Das Gerät umfasst ein spannungsführendes Element 320, welches über den Netzspannungsanschluss 310 ohne eine galvanische Kopplung betrieben wird. Ist das spannungsführende Element daher eingeschaltet, liegt an diesem Netzspannung an. Dabei sind zwei Netzspannungsleitungen 311 und 312 vorgesehen, welche das spannungsführende Element 320 mit Netzspannung versorgen.

Das erfindungsgemäße Gerät weist weiterhin ein Anwendungsteil 330 auf, mit welchem ein Anwender bei Betrieb des Gerätes in Berührung kommen kann. Das Anwendungsteil 330 ist dabei von dem spannungsführenden Element 320 durch eine Basisisolation 340 isoliert. Hierdurch wird ein Basisschutz gegen die Gefahr eines Stromschlages zur Verfügung gestellt. Das Anwendungsteil ist dabei im Ausführungsbeispiel elektrisch leitend, so dass bei einem Versagen der Basisisolation 340 die Gefahr eines Stromschlages für einen Benutzer besteht, welcher mit dem Anwendungsteil 330 in Kontakt kommt.

Das erfindungsgemäße Gerät wird dabei üblicherweise über einen Netzspannungsanschluss des öffentlichen Stromnetzes (z.B. einen 110 V bzw. 230 V Netzspannungsanschluss oder einem entsprechenden Drehstromanschluss) mit Strom versorgt. Eine der beiden Anschlussleitungen für den Wechselstrom ist daher geerdet. Kommt es nun zu einem Fehler in der Basisisolation, muss verhindert werden, dass ein Benutzer, welcher in Kontakt mit dem Anwendungsteil und der Erde steht, hierdurch einen gefährlichem Stromfluss ausgesetzt ist.

Hierfür ist erfindungsgemäß ein Isolationswächter 350 vorgesehen, welcher die Qualität der Basisisolation 340 des Anwendungsteils 330 gegenüber dem spannungsführenden Element 320 überwacht. Bei Über- bzw. Unterschreitung eines vorgegebenen Grenzwertes nimmt der Isolationswächter 350 dabei über die Schalter 360 und 370 eine Abschaltung der Stromversorgung des spannungsführenden Elementes 320 vor. Die ständige Überwachung der Qualität der Basisisolation erlaubt es so, auch ohne einen Schutzleiter einen sicheren Fehlerschutz zur Verfügung zu stellen.

Dabei steht der Isolationswächter 350 über eine Leitung 351 mit der ersten Spannungszuführung 311 des spannungsführenden Elementes 320 sowie mit einer zweiten Leitung 352 mit der zweiten Spannungszuführung 312 des spannungsführenden Elementes 320 in Verbindung. Die Verbindung erfolgt dabei bei dem in Figur 14a gezeigten Ausführungsbeispiel über eine Widerstandsbrücke. Weiterhin steht der Isolationswächter über eine Leitung 353 mit dem Anwenderteil 330 elektrisch leitend in Verbindung. Der Isolationswächter kann so ständig die Qualität der Basisisolation zwischen dem Anwenderteil 340 und dem spannungsführenden Element 320 überwachen.

In Figur 14b ist ein Ausführungsbeispiel eines spannungsführenden Elementes gezeigt. Dabei handelt es sich um ein keramisches Heizelement, bei welchem eine Widerstandsbahn 321 als spannungsführendes Element auf einer Keramikplatte 341 angeordnet ist, welche als Basisisolation zu der auf der Keramikplatte 341 angeordneten Heizplatte 331 dient. Bei der Heizplatte 331 handelt es sich vorteilhafterweise um eine Aluminiumplatte. Die beiden Enden der Widerstandsbahn stehen dabei mit den Netzspannungsleitungen 311 und 312 in Verbindung.

Der Widerstandwächter steht nun über die Leitungen 351 und 352 mit den beiden Enden der Widerstandsbahn und über die Leitung 353 mit der Heizplatte 331 in Verbindung. Die Heizplatte 331 weist hierfür einen entsprechenden Anschluss 332 für die Leitung 353 auf. Der Isolationwächter kann so die Qualität der Isolation 341 zwischen der spannungsführenden Widerstandsbahn 321 und der Heizplatte 331 überwachen. Natürlich kann die vorliegende Erfindung aber auch bei anderen spannungsführenden Elementen eingesetzt werden.

Die Überwachung der Qualität der Isolation kann dabei auf unterschiedliche Art und Weise erfolgen. In den Figuren 15 und 16 sind dabei zwei solcher Überwachungs-Prinzipien schematisch dargestellt. Dabei ist jeweils wieder ein elektrisches Gerät mit einem spannungsführenden Element 320, einem Anwendungsteil 330 und einer Basisisolation 340 dargestellt. Die Basisisolation 340 ist in diesen Ausführungsbeispielen dabei als eine doppelte Isolation ausgeführt. Weiterhin ist wiederum ein Isolationswächter 350 gezeigt, welcher sowohl mit den Spannungszuführungen 311 und 312 des spannungsführenden Elements 320, als auch mit dem Anwendungsteil, 330 in Verbindung steht. Die Schaltungsanordnung zum Abschalten der Netzspannung bei Detektion eines Isolationsfehlers ist dabei in den Figuren 15 und 16 nicht dargestellt, kann jedoch wie in Figur 14 gezeigt, implementiert werden.

Die Spannungszuführung 312 des Netzspannungsanschlusses in Fig. 15 und 16 ist als neutrale Leitung N gekennzeichnet, die Spannungszuführung 312 ist als Phase P gekennzeichnet. Weiterhin ist in Figuren 15 und 16 jeweils eine Person gezeigt, welche z.B. über ihre Füße mit der Erde 380 in Verbindung steht. Berührt die Person dabei das Anwendungsteil 330, und ist die Basisisolation 340 defekt, so könnte ohne den Isolationswächter 350 ein gefährlich hoher Strom von der Phase P des Netzanschlusses über das Anwendungsteil und die Bedienperson zur Erde 380 fließen. Der erfindungsgemäße Isolationswächter überwacht jedoch die Qualität der Basisisolation ständig und schaltet die Stromversorgung des spannungsführenden Teils 320 ab, wenn er eine fehlerhafte Isolation erkennt. So kann ein gefährlicher Stromschlag verhindert werden.

In Figur 15 ist dabei ein passiver Isolationswächter vorgesehen, welcher jeweils den Stromfluss zwischen den Spannungszuführungen 311 und 312 und dem Anwendungsteil 330 bestimmt. Übersteigt dieser Stromfluss einen gewissen Grenzwert, so schaltet der Isolationswächter die Stromversorgung ab.

In Figur 16 ist dagegen eine aktive Überwachung der Basisisolation 340 vorgesehen. Dabei legt der Isolationswächter 350 ein Spannungssignal jeweils an die Spannungszuführungen 311 bzw. 312 und an das Anwendungsteils 330, und misst den hierdurch erzeugten Stromfluss. Hierdurch kann der Isolationswiderstand bestimmt werden.

Bei dem erfindungsgemäßen Gerät handelt es sich vorteilhafterweise um ein Gerät mit einem Gehäuse, welches über ein Netzkabel an einem Netzspannungsanschluss angeschlossen werden kann. Insbesondere kann das Gerät dabei vorteilhafterweise über einen üblichen zweipoligen Netzstecker an einer Steckdose angeschlossen werden, da kein Schutzleiteranschluss benötigt wird. Besonders vorteilhaft kommt die vorliegende Erfindung dabei bei medizinischen Geräten zum Einsatz.

In einer besonders vorteilhaften Ausführungsform handelt es sich bei dem erfindungsgemäßen Gerät dabei um ein Dialysegerät, wie es bereits weiter vorne anhand der Figuren 1 bis 13 näher dargestellt wurde. Vorteilhafterweise handelt es sich dabei bei dem spannungsführenden Element 320 um ein Heizelement. Insbesondere handelt es sich dabei um ein keramisches Heizelement, wie dies bereits weiter vorne beschrieben wurde. Bei dem Anwendungsteil 330 handelt es sich dann vorteilhafterweise um eine Heizplatte, wie sie ebenfalls bereits weiter vorne beschrieben wurde. Insbesondere kann diese Heizplatte 330 dabei mit einem Heizbereich des Fluidsystems in Kontakt gebracht werden, um das Dialysat aufzuwärmen.

Die vorliegende Erfindung erlaubt es, ein Dialysegerät ohne Schutzleiter zu betreiben, und dennoch das bzw. die Heizelemente ohne Zwischenschaltung eines Trenntransformators am Netz zu betreiben. Der Isolationswächter stellt dabei sicher, dass eine Bedienperson selbst bei einem Ausfall der Basisisolation zwischen dem Heizelement und der Heizplatte nicht durch einen elektrischen Schlag gefährdet werden kann.

Neben dem Abschalten der Stromversorgung für das spannungsführende Element kann der Isolationswächter vorteilhafterweise zudem die Gerätesteuerung informieren, dass die Basisisolation fehlerhaft ist. Insbesondere kann dabei eine Anzeige erfolgen, welche die Bedienperson auf die Fehlerhaftigkeit der Basisisolation aufmerksam macht. Alternativ kann jedoch auch die Stromversorgung des gesamten Gerätes abgeschaltet werden.

Weiterhin kann das erfindungsgemäße einen Initialtest aufweisen, welcher die Funktion des Isolationswächters beim Einschalten des Gerätes durch Überbrücken der Basisisolierung z.B. über einen Schalter und einen Widerstand überprüft. Hierdurch wird eine zusätzliche Sicherheit hinsichtlich der korrekten Funktion gewährleistet.

In Figur 17 ist nun ein Ausführungsbeispiel eines Heizmoduls gemäß dem zweiten Aspekt der vorliegenden Erfindung näher dargestellt. Das Heizmodul 400 umfasst eine Heizplatte 401, auf welcher zwei Heizelemente 410 und 420 aufgebracht sind. Alternativ könnte dabei selbstverständlich auch nur ein einzelnes Heizelement eingesetzt werden, oder mehr als zwei Heizelemente.

Die Heizelemente weisen dabei jeweils ein Heizwendel auf, welcher auf einer keramischen Schicht aufgebracht ist. Das Heizelement 410 weist dabei den Heizwendel 412 auf, welcher auf einer keramischen Trägerplatte 411 aufgebracht ist.

Figur 17 zeigt dabei das Heizmodul in der oberen Darstellung in einer Ansicht von hinten und in der unteren Darstellung in einer Seitenansicht. Dabei ist der Schichtaufbau des Heizmoduls mit der Heizplatte 401 und der keramischen Trägerplatte 411 für die Heizwendel zu erkennen.

Erfindungsgemäß besteht nun auch die Heizplatte 401, auf welcher die Heizelemente angeordnet sind, aus einem keramischen Material. Hierdurch wird gegenüber den im Stand der Technik eingesetzten Heizplatten aus Aluminium eine zusätzliche elektrische Isolierung des Heizmoduls und damit des medizinischen Gerätes erreicht.

Im Ausführungsbeispiel besteht die die erste keramische Schicht bildende Trägerplatte 411 der Heizelemente, auf welcher der Heizwendel 412 angeordnet ist, aus Aluminiumoxid. Die Heizplatte 401 besteht im Ausführungsbeispiel dagegen aus Aluminiumnitrid. Beide Materialien haben sehr gute Wärmeleiteigenschaften bei gleichzeitig sehr guten Isolationseigenschaften.

Die keramischen Trägerplatten der Heizelemente sind dabei über einen Wärmekleber auf der Heizplatte 401 aufgeklebt. Als Wärmeleitkleber wird vorteilhafterweise ein Silikonkleber eingesetzt.

Im Ausführungsbeispiel weist die Trägerplatte 411 der Heizelemente eine Stärke von 1,0 mm auf, die Heizplatte 401 eine Stärke von 1,5 mm. Die Schichtdicke des Wärmeleitklebers kann zwischen 0,1 mm und 0,5 mm betragen.

Die Heizelemente weisen zum Anschluß an die Stromversorgung 440 Anschlüsse 413 auf, mit welchen Anschlußleitungen verbunden werden können. Vorteilhafterweise stehen die Anschlußelemente 413 dabei rückseitig von den keramischen Trägerplatten 411 weg. Weiterhin können die Heizelemente jeweils mit einem Temperatursensor 414 ausgestattet sein. Die Temperatursensoren 414 können ebenfalls eine Widerstandsbahn umfassen, wobei hier Anschlüsse 415 zum Anschluß an eine Auswerteelektronik vorgesehen sind.

Die beiden Heizelemente 410 und 420 können über eine Verbindungsleitung 430 miteinander verbunden werden, so dass beide Heizelemente seriell geschaltet sind. Alternativ wäre jedoch auch eine Parallelschaltung denkbar. Vorteilhafterweise ist dabei in die Verbindungsleitung 430 eine Temperatursicherung integriert.

Erfindungsgemäß können zwei oder mehr identische Heizelemente eingesetzt werden. Insbesondere können zwei oder mehr Heizelemente nenbeinander auf einer gemeinsamen Heizplatte 401 angeordnet werden.

Vorteilhafterweise ragt die Heizplatte 401 dabei in ihren Randbereichen über die Trägerplatten 411 der Heizelemente hinaus. Weiterhin vorteilhafterweise weisen die jeweiligen keramischen Platten 411 und 401 keine Durchbrechungen auf.

Zur Stabilisieurng des gesamten Heizmoduls kann zusätzlich die Heizplatte 401 auf einen Rahmen geklebt werden. Weiterhin kann vorgesehen sein, dass das gesamte Heizmodul in das Gehäuse des medizinischen Gerätes einvulkanisiert wird, insbesondere in die Schublade oder den Maschinenblock.

Das erfindungsgemäße Heizmodul kann in gleicher Weise eingesetzt werden wie die bereits vorne in Abschnitt 2.1 dargestellte Heizung. Insbesondere wird das Heizmodul wie dort beschrieben zum Beheizen von Dialysat eingesetzt. Vorteilhafterweise kann dabei die Heizplatte 401 direkt mit den Fluidwegen der Dialysemaschine gekoppelt werden, beispielsweise mit einem Heizbereich einer Kassette oder mit einem Heizbeutel.

Selbstverständlich kann ein Heizmodul gemäß dem zweiten Aspekt der vorliegenden Erfindung auch mit einem Isolationswächter kombiniert werden, wie er weiter vorne mit Bezug auf die Figuren 14 und 16 näher dargestellt wurde. Insbesondere kann das erfindungsgemäße Heizmodul dabei anstelle des in Figur 14b dargestellten Heizmoduls eingesetzt werden. Der Anschluß der Leitung 353 erfolgt dann aber selbstverständlich nicht an der keramischen Heizplatte 401, welche nicht leitend ist, sondern an einem leitenden Gehäuseelement. Hierdurch könnte eine nochmals erhöhte Sicherheit erreicht werden.

Erfindungsgemäß erlaubt jedoch auch der zweite Aspekt der vorliegenden Erfindung für sich allein, auf einen Schutzleiter zu verzichten und dennoch das Heizmodul ohne Zwischenschaltung eines Trenntransformators am Netz zu betreiben. Die durch die keramische Heizplatte 401 gebildete zweite Isolationsschicht stellt dabei sicher, dass eine Bedienperson selbst beim Ausfall der Basisisolation zwischen dem Heizwendel und der Heizplatte nicht durch einen elektrischen Schlag gefährdet werden kann.

Die vorliegende Erfindung ermöglicht so in beiden Aspekten unabhängig voneinander einen Fehlerschutz für die Basisisolierung, welcher sicherstellt, dass auch bei einer defekten Basisisolierung keine Gefahr für einen Anwender ausgeht.

## Patentansprüche

1. Elektrisch über einen Netzspannungsanschluss betreibbares medizinisches Gerät (300), mit einem spannungsführenden Element (320) und einem Anwendungsteil (330),
wobei das Anwendungsteil (330) gegenüber dem spannungsführenden Element (320) durch eine Basisisolation (340) isoliert ist und wobei ein Isolationswächter (350) vorgesehen ist, welcher die Qualität der Basisisolation des Anwendungsteiles r (330) gegenüber dem spannungsführenden Elemente (320) überwacht, **dadurch gekennzeichnet,**
**dass** der Isolationswächter (350) einen Stromfluss und/oder Widerstand zwischen
dem spannungsführenden Element (320)
und dem Anwendungsteil (330) bestimmt.

2. Gerät nach Anspruch 1, wobei es sich bei dem medizinischen Gerät um ein Dialysegerät handelt.

3. Gerät nach Anspruch 1 oder 2, wobei der Isolationswächter auf einem aktiven Messprinzip beruht und ein Spannungssignal zwischen dem spannungsführenden Element und dem Anwendungsteil anlegt und den daraus resultierenden Stromfluss bestimmt.

4. Gerät nach einem der vorangegangenen Ansprüche, wobei der Isolationswächter jeweils den Stromfluss und/oder Widerstand zwischen dem Anwendungsteil und einer ersten und den Stromfluss und/oder Widerstand zwischen dem Anwendungsteil und einer zweiten Spannungszuführung (312) des spannungsführenden Elementes (320) bestimmt.

5. Gerät nach einem der vorangegangenen Ansprüchen, wobei das spannungsführende Element ohne eine galvanische Trennung an der Netzspannung betrieben wird.

6. Gerät nach einem der vorangegangenen Ansprüchen, wobei der Isolationswächter die Stromversorgung des spannungsführenden Elements abschaltet, wenn er eine defekte Basisisolation erkennt.

7. Gerät nach einem der vorangegangenen Ansprüchen, wobei die Gerätesteuerung eine Funktion zum Testen der ordnungsgemäßen Funktion des Isolationswächters aufweist.

8. Gerät nach einem der vorangegangenen Ansprüchen, wobei es sich bei dem spannungsführenden Element um ein Heizelement handelt.

9. Gerät nach einem der vorangegangenen Ansprüchen, wobei es sich bei dem Anwendungsteil um ein Gehäuseelement handelt.

10. Gerät nach einem der vorangegangenen Ansprüchen, wobei es sich bei dem Anwendungsteil um eine Heizplatte handelt.

11. Gerät nach Anspruch 10, wobei es sich bei dem medizinischen Gerät um ein Dialysegerät handelt und wobei das spannungsführende Element ein keramisches Heizelemente und das Anwendungsteil eine Heizplatte darstellt, welche von der Keramikschicht des keramischen Heizelementes gegeneinander isoliert sind, wobei über das Heizelement Dialysat beheizt werden kann und wobei ein Heizbereich eines Fluidsystems an die Heizplatte ankoppelbar ist.

12. Gerät nach einem der vorangegangenen Ansprüchen, wobei das Gerät keinen Schutzleiteranschluss aufweist.

13. Verfahren zum Betrieb eines elektrischen Gerätes über einen Netzspannungsanschluss, zum Betrieb eines medizinischen Gerätes nach einem der vorangegangenen Ansprüchen, wobei das Gerät ein spannungsführendes Element und ein Anwendungsteil aufweist, wobei das Anwendungsteil von dem spannungsführenden Element durch eine Basisisolation isoliert ist, **dadurch gekennzeichnet,**
**dass** die Qualität der Basisisolation des Anwendungsteiles gegenüber dem spannungsführenden Element überwacht wird und der Isolationswächter einen Stromfluss und/oder Widerstand zwischen dem spannungsführenden Element und dem Anwendungsteil bestimmt.

## Claims

1. A medical device (300) operable electrically via a mains voltage connection, having a live element (320) and an application part (330), wherein the application part (330) is insulated by a basic insulation (340) with respect to the live element (320) and wherein an insulation monitor (350) is provided which monitors the quality of the basic insulation of the application part (330) with respect to the live element (320),
**characterized in that**
the insulation monitor (350) determines a current flow and/or a resistance between the live element (320) and the application part (330).

2. A device in accordance with claim 1, wherein the medical device is a dialysis device.

3. A device in accordance with claim 1 or claim 2, wherein the insulation monitor is based on an active measuring principle and applies a voltage signal between the live element and the application part and determines the current flow resulting therefrom.

4. A device in accordance with one of the preceding claims, wherein the insulation monitor determines the current flow and/or the resistance between the application part and a first voltage feed and the current flow and/or the resistance between the application part and a second voltage feed (312) respectively of the live element (320).

5. A device in accordance with one of the preceding claims, wherein the live element is operated without a galvanic isolation at the mains voltage.

6. A device in accordance with one of the preceding claims, wherein the insulation monitor switches the power supply of the live element off when it recognizes a defective basic insulation.

7. A device in accordance with one of the preceding claims, wherein the device controller has a function for testing the proper function of the insulation monitor.

8. A device in accordance with one of the preceding claims, wherein the live element is a heating element.

9. A device in accordance with one of the preceding claims, wherein the application part is a housing element.

10. A device in accordance with one of the preceding claims, wherein the application part is a heating plate.

11. A device in accordance with claim 10, wherein the medical device is a dialysis device and wherein the live element constitutes a ceramic heating element and the application part constitutes a heating plate which are insulated from each other by the ceramic layer of the ceramic heating element, wherein dialysate can be heated via the heating element and wherein a heating zone of a fluid system can be coupled to the heating plate.

12. A device in accordance with one of the preceding claims, wherein the device does not have a protective conductor terminal.

13. A method for operating an electrical device via a mains voltage connection, for operating a medical device in accordance with one of the preceding claims, wherein the device has a live element and an application part, wherein the application part is insulated from the live element by a basic insulation, **characterized in that**
the quality of the basic insulation of the application part is monitored with respect to the live element and the insulation monitor determines a current flow and/or a resistance between the live element and the application part.

## Revendications

1. Appareil médical (300) électrique pouvant fonctionner sur secteur, comprenant un élément conducteur de tension (320) et une partie application (330),
la partie application (330) étant isolée par rapport à l'élément conducteur de tension (320) par une isolation principale (340) et un contrôleur d'isolement (350) étant prévu, qui surveille la qualité de l'isolation principale de la partie application (330) par rapport à l'élément conducteur de tension (320), **caractérisé en ce que**
le contrôleur d'isolement (350) détermine un flux de courant et/ou une résistance entre l'élément conducteur de tension (320) et la partie application (330).

2. Appareil selon la revendication 1, dans lequel l'appareil médical est un appareil de dialyse.

3. Appareil selon la revendication 1 ou 2, dans lequel le contrôleur d'isolement est basé sur un principe de mesure actif et applique un signal de tension entre l'élément conducteur de tension et la partie application et détermine le flux de courant en résultant.

4. Appareil selon l'une des revendications précédentes, dans lequel le contrôleur d'isolement détermine respectivement le flux de courant et/ou la résistance entre la partie application et une première alimentation en tension et le flux de courant et/ou la résistance entre la partie application et une seconde alimentation en tension (312) de l'élément conducteur de tension (320).

5. Appareil selon l'une des revendications précédentes, dans lequel l'élément conducteur de tension fonctionne sur le secteur sans séparation électrique.

6. Appareil selon l'une des revendications précédentes, dans lequel le contrôleur d'isolement coupe l'alimentation électrique de l'élément conducteur de tension lorsqu'il détecte une isolation principale défectueuse.

7. Appareil selon l'une des revendications précédentes, dans lequel la commande de l'appareil comporte une fonction de test du fonctionnement correct du contrôleur d'isolement.

8. Appareil selon l'une des revendications précédentes, dans lequel l'élément conducteur de tension est un élément chauffant.

9. Appareil selon l'une des revendications précédentes, dans lequel la partie application est un élément de boîtier.

10. Appareil selon l'une des revendications précédentes, dans lequel la partie application est une plaque chauffante.

11. Appareil selon la revendication 10, dans lequel l'appareil médical est un appareil de dialyse et dans lequel l'élément conducteur de tension constitue un élément chauffant en céramique et la partie application une plaque chauffante, qui sont isolés l'un par rapport à l'autre par la couche en céramique de l'élément chauffant en céramique, dans lequel du dialysat peut être chauffé par le biais de l'élément chauffant et dans lequel une zone de chauffage d'un système fluidique peut être couplée à la plaque chauffante.

12. Appareil selon l'une des revendications précédentes, dans lequel l'appareil ne comporte aucune borne de terre de protection.

13. Procédé de fonctionnement d'un appareil électrique sur secteur, destiné au fonctionnement d'un appareil médical selon l'une des revendications précédentes, l'appareil comportant un élément conducteur de tension et une partie application, la partie application étant isolée de l'élément conducteur de tension par une isolation principale,
**caractérisé en ce que**
la qualité de l'isolation principale de la partie application par rapport à l'élément conducteur de tension est surveillée et le contrôleur d'isolement détermine un flux de courant et/ou une résistance entre l'élément conducteur de tension et la partie application.
